# EUROPEAN PATENT APPLICATION

(11) **EP 4 610 250 A1**
(43) Date of publication of application: **03.09.2025**
(21) Application number: 23880901.6
(22) Date of filing: 03.08.2023
(51) Int. Cl.: C07C 237/22, A61K 31/00

(54) **GPR139 RECEPTOR AGONIST, PREPARATION METHOD THEREFOR, AND APPLICATION THEREOF**

(30) Priority: 28.10.2022 CN 202211338536
(71) Applicant: Neubo Apptech Co., Ltd., Ningbo, Zhejiang 315100 (CN)
(72) Inventor: CHEN, Jingcai, Ningbo, Zhejiang 315100 (CN)
(74) Representative: dompatent von Kreisler Selting Werner - Partnerschaft von Patent- und Rechtsanwälten mbB
(86) International application number: PCT/CN2023/111008
(87) International publication number: WO 2024/087782

(57) **Abstract**

Provided are a compound as shown in formula (I), or a pharmaceutically acceptable salt thereof. In addition, also provided is a preparation method for the compound and the pharmaceutically acceptable salt thereof, and an application of the compound in the preparation of a medicine for treating or preventing GPR139 receptor related diseases.

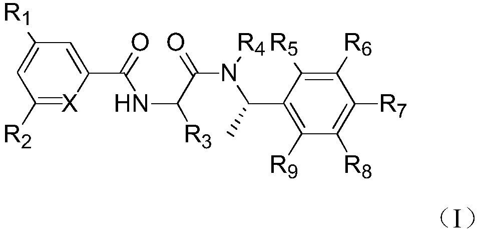

## Description

### TECHNICAL FIELD

The present invention relates to the field of medicinal chemistry. Specifically, the present invention relates to a ligand molecule for G protein-coupled receptor 139 (GPR139).

### BACKGROUND

G protein-coupled receptor 139 (GPR139) was first discovered by Gloriam et al. in 2005 (Biochim Biophys Acta 2005; 1722:235-246), also known as GPCR12, PGR3, KOR3L, GPRg1. GPR139 is mainly expressed in the central nervous system (CNS), including the habenula, striatum, thalamus, hypothalamus, and pituitary gland. Its amino acid sequence is highly conserved in different species. For example, the homology of the human GPR139 protein sequence with mice, chickens, and zebrafish is 96%, 92%, and 70%, respectively. When GPR139 receptor is expressed on the cell surface, it consists of an N-terminal fragment, 7 transmembrane fragments, and a C-terminal fragment. The N-terminal fragment is composed of 26 amino acids and contains one cysteine. Wang et al. reported that GPR139 receptor and dopamine receptor D2 are co expressed in different tissues of mouse brain, and in vitro cell experiments confirmed that GPR139 can promote the generation of calcium flow signals by dopamine receptor D2 agonists, which can be inhibited by both dopamine receptor D2 antagonists and GPR139 antagonists (Frontiers in Neuroscience, March 2019, Volume 13, Article 281). In addition, Nepomuceno et al. confirmed that GPR139 can synergize with melanocortin receptors 3 or 5 (MC3/MC5). These results indicate that GPR139 can form heterodimers with other receptors on the cell surface and exert physiological effects. In addition, our experiments have confirmed that GPR139 receptors can form homodimers through N-terminal cysteine.

While different research teams are searching for ligands for GPR139, including agonists and antagonists, Liu et al. reported that L-phenylalanine and L-tryptophan are endogenous agonists of GPR139 (Mol Pharmacol 2015; 88:911-925).

Existing research indicates that GPR139 receptors have important physiological functions and are potential drug targets for anti anxiety, anti depression, anti schizophrenia, treatment of Parkinson's syndrome, treatment of drug and alcohol abuse, treatment of autism, treatment of epilepsy, treatment of mania, and treatment of metabolic related diseases. The structural, expression, pharmacological, and physiological functional information of GPR139 provides a solid foundation for the design and synthesis of novel GPR139 agonists.

### SUMMARY OF THE INVENTION

In the first aspect of the present invention, it provides a compound as shown in formula (I), a deuterated compound thereof, or a pharmaceutically acceptable salt thereof, 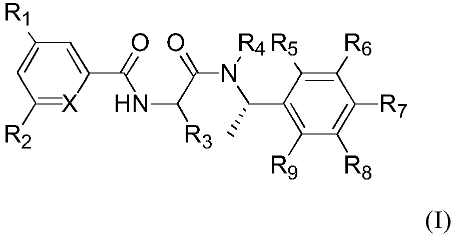 wherein,
X is selected from the group consisting of: N, CH;
R₁ and R₂ are each independently selected from the group consisting of: hydrogen, halogen, hydroxyl, carboxyl, C₁₋₃ alkyl, C₁₋₆ acyl, C₁₋₃ alkyl-carbonyl-C₁₋₃ alkyl, C₁₋₆ alkyl-hydroxyl, C₁₋₆ alkyl-aldehyde, C₁₋₆ alkyl, C₁₋₆ haloalkyl, C₁₋₆ alkoxy, and C₁₋₆ haloalkoxy;
R₃ is selected from the group consisting of: hydrogen, halogen, hydroxyl, carboxyl, C₁₋₆ acyl, C₁₋₃ alkyl-carbonyl-C₁₋₃ alkyl, C₁₋₆ alkyl-hydroxyl, C₁₋₆ alkyl-aldehyde, C₁₋₆ alkyl, C₁₋₆ haloalkyl, C₁₋₆ alkoxy, C₁₋₆ haloalkoxy, C₃₋₈ saturated or unsaturated carbocyclyl, 3-10 membered saturated or unsaturated heterocyclyl, C₆₋₁₀ aryl, 5-12 membered heteroaryl;
R₄ is selected from the group consisting of: hydrogen, halogen, carboxyl, C₁₋₆ acyl, C₁₋₃ alkyl-carbonyl-C₁₋₃ alkyl, C₁₋₆ alkyl-hydroxyl, C₁₋₆ alkyl-aldehyde, C₁₋₆ alkyl, C₁₋₆ haloalkyl;
R₅, R₆, R₇, R₈, and R₉ are each independently selected from the group consisting of: hydrogen, halogen, hydroxyl, carboxyl, C₁₋₆ acyl, C₁₋₃ alkyl-carbonyl-C₁₋₃ alkyl, C₁₋₆ alkyl-aldehyde, C₁₋₆ alkyl, C₁₋₆ haloalkyl, C₁₋₆ alkoxy, C₁₋₆ haloalkoxy;
wherein, the carbocyclyl, heterocyclyl, aryl, or heteroaryl groups may be monocyclic, spirocyclic, fused-ring, or bridged-ring structural groups.

In some embodiments, R₁ and R₂ are independently selected from the group consisting of: hydrogen, halogen, hydroxyl, carboxyl, C₁₋₃ alkyl, C₁₋₃ alkoxy, C₁₋₃ haloalkyl, and C₁₋₃ haloalkoxy.

In another preferred embodiment, R₁ and R₂ are each independently selected from the group consisting of: hydrogen, chlorine, fluorine, -CH₃, -CF₃, -OCH₃.

In some embodiments, R₃ is selected from the group consisting of: hydrogen, halogen, hydroxyl, carboxyl, C₁₋₃ acyl, C₁₋₃ alkyl-carbonyl-C₁₋₃ alkyl, C₁₋₃ alkyl-hydroxyl, C₁₋₃ alkyl-aldehyde, C₁₋₃ alkyl, C₁₋₃ haloalkyl, C₁₋₃ alkoxy.

In another preferred embodiment, R₃ is selected from the group consisting of: hydrogen, -CH₂OH.

In some embodiments, R₄ is selected from the group consisting of: hydrogen, C₁₋₃ alkyl, C₁₋₃ haloalkyl, C₃₋₆ saturated or partially unsaturated carbocyclyl, 3-10 membered saturated or partially unsaturated heterocyclyl.

In another preferred embodiment, R₄ is hydrogen or methyl.

In some embodiments, R₅, R₆, R₇, R₈, and R₉ are each independently selected from the group consisting of: hydrogen, halogen, hydroxyl, carboxyl, C₁₋₃ alkyl, C₁₋₃ alkoxy, C₁₋₃ haloalkyl, C₁₋₃ haloalkoxy.

In another preferred embodiment, R₅, R₆, R₇, R₈, and R₉ are each independently selected from the group consisting of: hydrogen, fluorine, chlorine.

In some embodiments the compound is selected from the group consisting of:

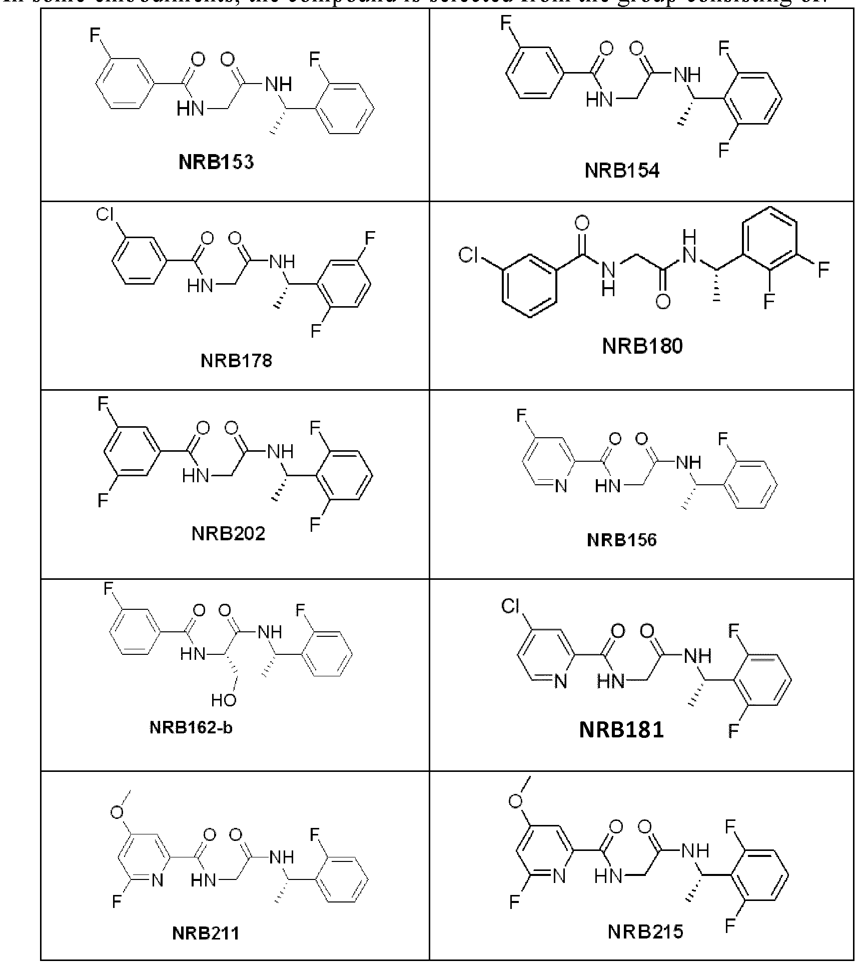

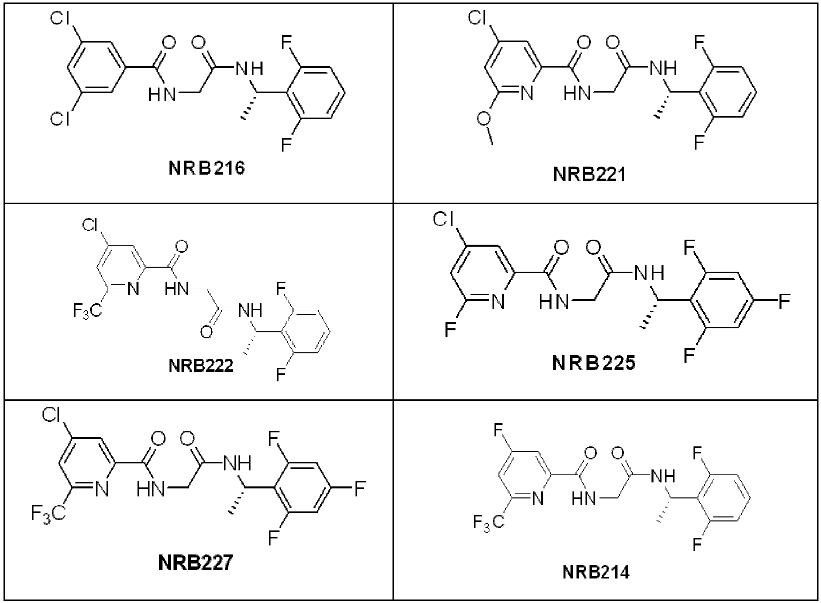

In the second aspect of the present invention, it provides a method for preparing the compound of the first aspect of the present invention or a pharmaceutically acceptable salt thereof, comprising the steps of: 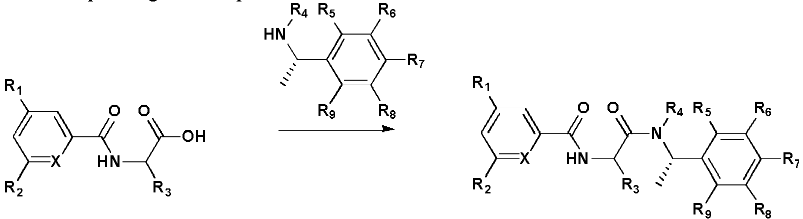 or 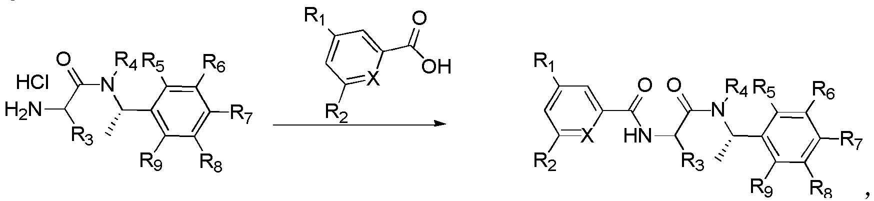 wherein, the definitions of X, R₁, R₂, R₃, R₄, R₅, R₆, R₇, R₈, and R₉ are as described earlier.

In the third aspect of the present invention, it provides a pharmaceutical composition, comprising (1) the compound of the first aspect of the present invention, a deuterated compound thereof, or a pharmaceutically acceptable salt thereof; and (2) pharmaceutically acceptable carriers.

In the fourth aspect of the present invention, it provides a method for preparing a pharmaceutical composition of the third aspect of the present invention, comprising the steps of: mixing the compound of the first aspect of the present invention, a deuterated compound thereof, or a pharmaceutically acceptable salt thereof, with a pharmaceutically acceptable carrier to form a drug mixture.

In the fifth aspect of the present invention, it provides a use of the compound of the first aspect of the present invention, a deuterated compound thereof, or a pharmaceutically acceptable salt thereof, or the pharmaceutical composition of the third aspect of the present invention, for the preparation of a medicament for treating or preventing a GPR139 receptor-associated disorder.

In another preferred embodiment, the GPR139 receptor-associated disorder comprises anxiety disorders, depression, mania, Parkinson's syndrome, drug and alcohol abuse, schizophrenia, autism, seizure, and epilepsy.

It is to be understood that, within the scope of the present invention, any technical features described above in the present invention may be combined with each other and with the technical features specifically described below (e.g., in the examples), thereby forming new or preferred technical solutions. Due to space limitations, we will not elaborate on each one here.

### BRIEF DESCRIPTION OF THE DRAWINGS

FIG.1 shows the detection of corticosterone levels in acutely restrained mice administered with different concentrations of compound NRB181 (**: p<0.01 for compound group vs. control group; ***: p<0.001 for compound group vs. control group).
FIG.2 shows the changes in sleep time of acutely restrained mice administered with different concentrations of compound NRB181 (**: p<0.01 for compound group vs. control group).
FIG.3 shows the effect of compound NRB181 on immobility time in the mouse forced swimming tests (**: p<0.01 for compound group vs. control group).
FIG.4 shows the effect of compound NRB181 on body weight changes in LPS induced mouse depression model (*: p<0.05 for compound + LPS group vs. placebo + LPS group; ***: p<0.001 for placebo + LPS group vs. placebo + saline group).
FIG.5 shows the effect of compound NRB181 on immobility time in the mouse forced swimming test in LPS induced mouse depression models (*: p<0.05 for compound + saline group vs. placebo + saline group; **: p<0.01 for compound + LPS group vs. placebo + LPS group).
FIG.6 shows the effect of compound NRB181 on the number of line crossings of mice in an Open Field Test in a LPS induced mouse depression model (*: p<0.05 for compound + LPS group vs. placebo + LPS group; ***: p<0.001 for placebo + LPS group vs. placebo + saline group).
FIG.7 shows the effect of compound NRB181 on the total distance traveled by mice in an open field test in a LPS induced mouse depression model (**: p<0.01 for compound + LPS group vs. placebo + LPS group; **: p<0.001 for placebo + LPS group vs. placebo + saline group)
FIG.8 shows the effect of compound NRB181 on the expression level of corticosterone in LPS induced mouse depression model (**: compound + LPS group vs. placebo + LPS group; *: p<0.05 for placebo + LPS group vs. placebo + saline group)

### DETAILED DESCRIPTION OF THE PREFERRED EMBODIMENTS

After extensive and in-depth research, the inventor unexpectedly discovered that the compound of the present invention has excellent GPR139 receptor agonist activity, and thus conducted a series of synthesis and biological activity tests. On this basis, the present invention has been completed.

The experimental methods in the following examples of the present invention that do not specify specific conditions are usually carried out under conventional conditions or conditions recommended by the manufacturer. The various commonly used chemical reagents used in the examples are all commercially available products.

Unless otherwise defined, all technical and scientific terms used in the present invention have the same meanings as those commonly understood by those skilled in the art belonging to the present invention. The terms used in the specification of the present invention are only for the purpose of describing specific examples and are not intended to limit the present invention.

### Terms

The terms "comprising," "having," and any grammatical variants thereof as used in the present invention are intended to encompass non-exclusive inclusion. For example, a process, method, apparatus, product, or equipment that includes a series of steps is not limited to the listed steps or modules, but may optionally also include steps that are not listed, or may optionally include other steps inherent to these processes, methods, products, or equipment.

The term "multiple" mentioned in the present invention refers to two or more. 'And/or' describes the association relationship between related objects, indicating that there can be three types of relationships, for example, A and/or B, which can represent: A exists alone, A and B exist simultaneously, and B exists alone. The character "/" generally indicates that the associated object before and after is an "or" relationship.

In the compounds of the present invention, when any variable occurs more than once in any constituent, each occurrence is defined independently of every other occurrence.
Similarly, combinations of substituents and variables are allowed as long as they stabilize the compound. A line drawn from a substituent into the ring system indicates that the designated bond may be attached to any substitutable ring atom. If the ring system is polycyclic, it means that this bond is only connected to any suitable carbon atom adjacent to the ring. It should be understood that ordinary technicians in this field can choose the substituents and substitution forms of the compounds of the present invention to provide chemically stable compounds that can be easily synthesized from readily available raw materials through the techniques in this field and the methods proposed below. If the substituent itself is replaced by more than one functional group, it should be understood that these functional groups can be on the same carbon atom or different carbon atoms, as long as the structure is stabilized.

The term "alkyl" used herein refers to saturated fatty hydrocarbon groups including branched and straight chains with a specific number of carbon atoms. For example, the definition of "C1-C8" in "C1-C8 alkyl" includes functional groups with 1, 2, 3, 4, 5, or 8 carbon atoms arranged in a straight or branched chain. The term "cycloalkyl" refers to a monocyclic saturated fatty hydrocarbon group with a specific number of carbon atoms. For example, "cycloalkyl" comprises cyclopropyl, methyl-cyclopropyl, 2,2-dimethyl-cyclobutyl, 2-ethyl-cyclopentyl, cyclohexyl, and so on.

The term "alkoxy" used herein represents an alkyl-oxy group, wherein the definition of alkyl is as shown above.

As understood by those skilled in the art, the term "halogen" used herein refers to chlorine, fluorine, bromine, and iodine.

The term "haloalkyl" used herein refers to an alkyl group in which one or more hydrogen atoms are substituted by halogens, wherein the definition of alkyl is as defined above.

The term "halogenated alkoxy" used herein refers to an alkyl-oxy group in which one or more hydrogen atoms are substituted by halogens, where the definition of alkyl is as defined above.

### Active ingredient

In the present invention, it provides an active ingredient that can effectively agonizes GPR139 receptor. The active ingredient is a compound represented by general formula (I), which is effective in preventing, treating, and/or alleviating GPR139-related diseases.

Experiments have shown that the active ingredients of the present invention can effectively agonize GPR139 receptors, thereby preventing, treating, and/or alleviating GPR139-related diseases.

It should be understood that the active ingredients of the present invention comprise compounds represented by general formula (I), or pharmaceutically acceptable salts thereof, or prodrugs thereof. It should be understood that the active ingredients of the present invention further comprise crystalline forms, amorphous compounds, and deuterated derivatives of the compound represented by general formula (I).

The term "pharmaceutically acceptable salt" refers to a salt formed by the compound of the present invention with an acid or base that is suitable for use as a drug. Pharmaceutically acceptable salts comprise inorganic salts and organic salts. A preferred type of salt is the salt formed between the compound of the present invention and an acid. Suitable acids for salt formation include but are not limited to: inorganic acids such as hydrochloric acid, hydrobromic acid, hydrofluoric acid, sulfuric acid, nitric acid, phosphoric acid, etc; organic acids such as formic acid, acetic acid, trifluoroacetic acid, propionic acid, oxalic acid, malonic acid, succinic acid, fumaric acid, maleic acid, lactic acid, malic acid, tartaric acid, citric acid, picric acid, benzoic acid, methanesulfonic acid, ethanesulfonic acid, p-toluenesulfonic acid, benzenesulfonic acid, naphthalene sulfonic acid, etc; And amino acids such as proline, phenylalanine, aspartic acid, glutamic acid, etc. Another preferred type of salt is the salt formed by the compound of the present invention with a base, such as alkali metal salts (e.g. sodium or potassium salts), alkaline earth metal salts (e.g. magnesium or calcium salts), ammonium salts (e.g. lower alkyl alcohol ammonium salts and other pharmaceutically acceptable amine salts), such as methylamine salts, ethylamine salts, propylamine salts, dimethylamine salts, trimethylamine salts, diethylamine salts, triethylamine salts, tert butylamine salts, ethylenediamine salts, hydroxyethyl amine salts, dihydroxyethylamine salts, and amine salts formed from morpholine, piperazine, and lysine, respectively.

### Preparation Method

The following provides a more specific description of the preparation method of the compound with the structure of formula (I) of the present invention, but these specific methods do not constitute any limitation on the present invention. The compounds of the present invention can also optionally be conveniently prepared by combining various synthetic methods described in this specification or known in the art, and such combinations can be easily carried out by those skilled in the art to which the present invention belongs.

Usually, in the preparation process, each reaction is carried out in an inert solvent at room temperature to reflux temperature (such as 0°C~80°C, preferably 0°C~50°C). The reaction time is usually 0.1-60 hours, preferably 0.5-48 hours.

The following general preparation route can be used to synthesize compounds with the structure of formula (I) of the present invention. 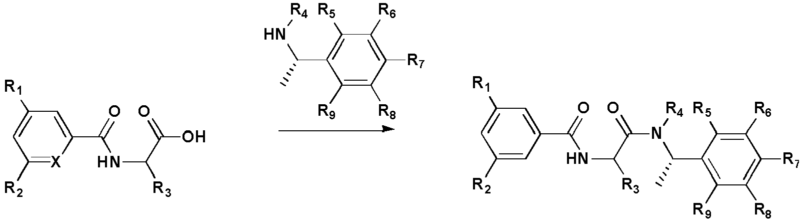 or

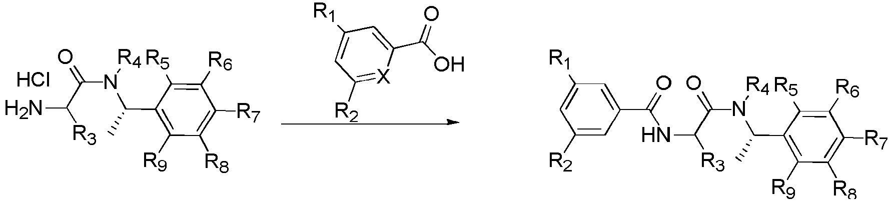

### Drug combination and administration method

Due to the excellent GPR139 receptor agonistic activity of the compounds of the present invention, the compounds of the present invention (including their various crystal forms), pharmaceutically acceptable inorganic or organic salts, hydrates or solvates, as well as pharmaceutical compositions comprising said compounds as the primary active ingredient, can be used for the treatment, prevention, and alleviation of GPR139 receptor-associated disorders.

The pharmaceutical composition of the present invention comprises the compound of the present invention or a pharmaceutically acceptable salt thereof in a safe and effective amount, and a pharmaceutically acceptable excipient or carrier. The term "safe and effective amount" refers to the amount of a compound that is sufficient to significantly improve the condition without causing serious side effects. Generally, pharmaceutical compositions contain 1-3000 mg (active dose range 3-30 mg/kg) of the compound of the present invention per dose, and more preferably 10-2000 mg of the compound of the present invention per dose. Preferably, the "one dose" is a capsule or tablet.

"Pharmaceutically acceptable carrier" refers to one or more compatible solid or liquid fillers or gel substances, which are suitable for human use and must have sufficient purity and sufficiently low toxicity. "Compatibility" here refers to the ability of each component in the composition to blend with the compounds of the present invention and with each other without significantly reducing the efficacy of the compounds. Examples of pharmaceutically acceptable carriers comprise cellulose and its derivatives (such as sodium carboxymethyl cellulose, sodium ethyl cellulose, cellulose acetate, etc.), gelatin, talc, solid lubricants (such as stearic acid, magnesium stearate), calcium sulfate, vegetable oils (such as soybean oil, sesame oil, peanut oil, olive oil, etc.), polyols (such as propylene glycol, glycerol, mannitol, sorbitol, etc.), emulsifiers (such as Tween^{®}), humectants (such as sodium dodecyl sulfate), coloring agents, flavoring agents, stabilizers, antioxidants, preservatives, pyrogen-free water, etc.

The administration method of the compound or pharmaceutical combination of the present invention is not particularly limited, and representative administration methods comprise (but are not limited to): oral, intratumoral, rectal, parenteral (intravenous, intramuscular, or subcutaneous), and topical administration.

Solid dosage forms for oral administration comprise capsules, tablets, pills, powders, and granules. In these solid dosage forms, the active compound is mixed with at least one conventional inert excipient (or carrier), such as sodium citrate or dicalcium phosphate, or with the following components: (a) fillers or compatibilizers, such as starch, lactose, sucrose, glucose, mannitol, and silica; (b) binders, such as hydroxymethylcellulose, alginate, gelatin, polyvinylpyrrolidone, sucrose, and arabic gum; (c) moisturizing agents, such as glycerin; (d) disinfectants, such as agar, calcium carbonate, potato starch or cassava starch, alginic acid, certain complex silicates, and sodium carbonate; (e) retarding agents, such as paraffin wax; (f) absorption accelerators, such as quaternary amine compounds; (g) humectants, such as cetyl alcohol and glycerol monostearate; (h) adsorbents, such as kaolin; and (i) lubricants, such as talc, calcium stearate, magnesium stearate, solid polyethylene glycol, sodium dodecyl sulfate, or mixtures thereof. Capsules, tablets, and pills may also comprise buffering agents.

Solid dosage forms such as tablets, sugar pills, capsules, pills, and granules can be prepared using coating and shell materials, such as casings and other materials known in the art. They may contain opacifiers, and the release of active compounds or compounds in this composition can be delayed in a certain part of the digestive tract. Examples of usable embedding components are polymeric substances and wax based substances. When necessary, the active compound can also form microcapsules with one or more of the above-mentioned excipients.

Liquid dosage forms for oral administration comprise pharmaceutically acceptable lotion, solutions, suspensions, syrups or tinctures. In addition to active compounds, liquid formulations may comprise inert diluents commonly used in this field, such as water or other solvents, solubilizers, and emulsifiers, such as ethanol, isopropanol, ethyl carbonate, ethyl acetate, propylene glycol, 1,3-butanediol, dimethylformamide, as well as oils, particularly cottonseed oil, peanut oil, corn germ oil, olive oil, castor oil, and sesame oil, or mixtures of these substances.

In addition to these inert diluents, the composition may further comprise adjuvants such as humectants, emulsifiers and suspensions, sweeteners, flavor Correctives, and flavorings.

In addition to active compounds, suspensions may comprise suspending agents such as ethoxylated isooctadecanol, polyoxyethylene sorbitol and dehydrated sorbitol esters, microcrystalline cellulose, methanol aluminum and agar, or mixtures of these substances.

Compositions for parenteral injection may comprise physiologically acceptable sterile aqueous or anhydrous solutions, dispersions, suspensions or lotion, and sterile powders for re dissolution into sterile injectable solutions or dispersions. Suitable aqueous and non-aqueous carriers, diluents, solvents, or excipients comprise water, ethanol, polyols, and their suitable mixtures.

The dosage forms of the compounds of the present invention for topical administration comprise ointments, powders, patches, sprays, and inhalants. The active ingredient is mixed under sterile conditions with a physiologically acceptable carrier, along with any preservatives, buffering agents, or propellants as required.

The compound of the present invention can be administered alone or in combination with other pharmaceutically acceptable compounds.

When using a pharmaceutical composition, a safe and effective amount of the compound of the present invention is administrated to a mammal in need of treatment (such as a human), wherein the dosage at the time of administration is the effective dosage considered in pharmacy. For a person weighing 60 kg, the daily dosage is usually 1~2000 mg, preferably 6~600 mg. Of course, the specific dosage should also consider factors such as the route of administration and the patient's health condition, which are within the skill range of skilled physicians.

**The main advantages of the present invention include:**
(a) The compounds of the present invention can efficiently agonize GPR139 receptors, with some compounds having EC50 values<0.1 µM.
(b) The compound of the present invention has simple synthesis and high yield.
(c) The compound of the present invention has low toxic side effects and good medicinal properties.

The present invention will be further explained in conjunction with specific examples. It should be understood that these examples are only used to illustrate the present invention and not to limit the scope of the present invention. The experimental methods without specific conditions specified in the following examples are usually carried out under conventional conditions or conditions recommended by the manufacturer. Unless otherwise specified, percentages and portions are calculated by weight. The starting materials used in the present invention are not specifically specified and are all commercially purchased.

### Examples

### Example 1 Preparation of compound NRB153

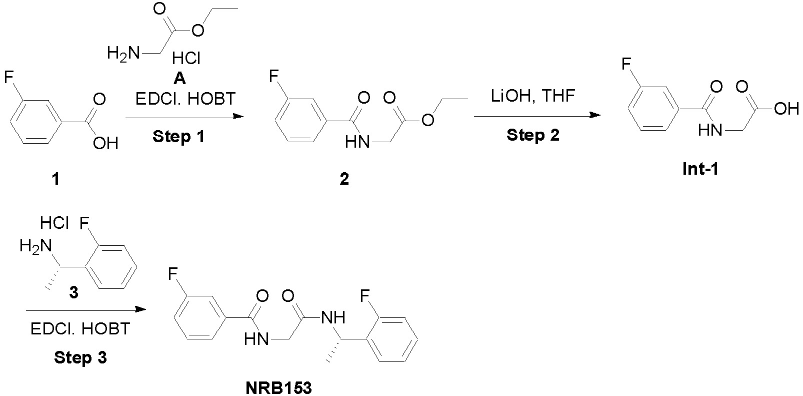

### Step 1:

Raw material 1 (1.5 g, 10.75 mmol) and raw material A (1 g, 7.16 mmol), EDCl (2.75 g, 14.33 mmol), HOBt (1.93 g, 14.33 mmol), and diisopropylethylamine (1.85 g, 14.33 mmol) were dissolved in 50 mL of dichloromethane. The mixture was stirred at room temperature overnight. The completion of the reaction was monitored by TLC. The aqueous solution (50 mL) was added, and the mixture was extracted with dichloromethane (3 × 50 mL). The organic phase was dried over anhydrous sodium sulfate, concentrated to dryness, and purified by column chromatography to obtain 1.6g of the product as a colorless oil. Analysis data: LCMS (ESI+): m/z 226 (M+H).

### Step 2:

Intermediate 2 (1.6 g, 7.11 mmol) was dissolved in tetrahydrofuran (20 mL) and water (20 mL), and LiOH.H₂O (896 mg, 21.33 mmol) was added. The reaction was stirred at room temperature for two hours. The completion of the reaction was monitored by TLC. After the reaction was completed, the pH was adjusted to 3-4 using a 1 mol/L dilute hydrochloric acid solution. The mixture was extracted with ethyl acetate (3 × 30 mL), and the organic phase was dried with anhydrous sodium sulfate. After concentration, 1.3 g of the product was obtained as a white solid. Analysis data: LCMS (ESI+): m/z 198 (M+H).

### Step 3:

Intermediate Int-1 (84 mg, 0.43 mmol) and (S) -1- (2-fluorophenyl) ethylamine hydrochloride (50 mg, 0.28 mmol), EDCl (109 mg, 0.57 mmol), HOBt (77 mg, 0.57 mmol), and diisopropylethylamine (74 mg, 0.571 mmol) were dissolved in 3 mL of dichloromethane and stirred at room temperature for four hours. The completion of the reaction was monitored by TLC. The reaction was quenched by adding an aqueous solution (5 mL) and then extracted with dichloromethane (3 × 3 mL). The organic phase was dried over anhydrous sodium sulfate. After concentration, the crude product was purified by preparative thin-layer chromatography (PTLC) to obtain 50 mg product as a white solid. Analysis data: LCMS (ESI+): m/z 319 (M+H); ¹HNMR (300 MHz, CDCl₃) δ 7.57-7.52 (m, 2H), 7.45-7.37 (m, 1H), 7.30-7.28 (m, 1H), 7.26-7.19 (m, 2H), 7.13-6.96 (m, 3H), 6.60-6.53 (m, 1H), 5.31 (q, J = 7.2 Hz, 1H), 4.18-4.12 (m, 2H), 1.54 (d, J = 7.2 Hz, 3H).

### Examples 2-5

The method of Example 1 was adopted, with the difference being that different substrates were used to replace Int-1 and/or (S) -1- (2-fluorophenyl) ethylamine hydrochloride, resulting in the preparation of compounds 2-5.

| NO. | Compound | Analysis data |
|---|---|---|
| 2 | 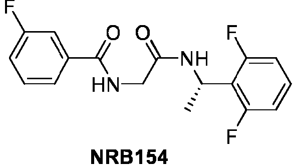 | LCMS (ESI+): m/z 337 (M+H); ¹HNMR (300 MHz, CDCl₃) *δ* 7.55-7.52 (m, 2H), 7.45-7.37 (m, 1H), 7.26-7.19 (m, 2H), 6.97 (brs, 1H), 6.91-6.85 (m, 2H), 6.59 (d, *J* = 8.1 Hz, 1H), 5.64 (q, *J* = 7.2 Hz, 1H), 4.18-4.10 (m, 2H), 1.55 (d, *J* = 7.2 Hz, 3H). |
| 3 | 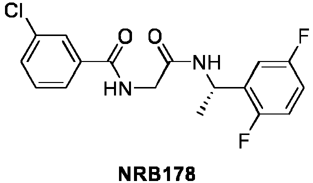 | LCMS (ESI+): m/z 353 (M+H); ¹HNMR (300 MHz, CD₃OD) *δ* 7.90 (s, 1H), 7.80 (d, *J* = 7.5 Hz, 1H), 7.55 (d, *J* = 7.8 Hz, 1H), 7.48-7.43 (m, 1H), 7.13-6.98 (m, 3H), 5.28-5.20 (m, 1H), 4.12-4.00 (m, 2H), 1.46 (d, *J* = 6.9 Hz, 3H). |
| 4 | 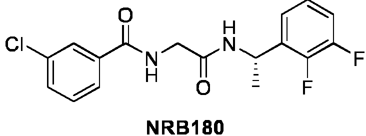 | LCMS (ESI+): m/z 353 (M+H); ¹HNMR (300 MHz, CD₃OD) *δ*□ 7.90 (s, 1H), 7.80 (d, *J* = 7.5 Hz, 1H), 7.55 (d, *J* = 7.8 Hz, 1H), 7.48-7.43 (m, 1H), 7.17-7.01 (m, 3H), 5.32-5.28 (m, 1H), 4.11-4.05 (m, 2H), 1.48 (d, *J* = 6.9 Hz, 3H). |
| 5 | 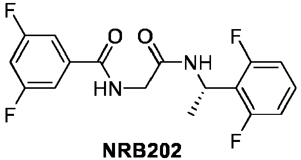 | LCMS (ESI+): m/z 355 (M+H); ¹H-NMR (300 MHz, CDCl₃) *δ* 7.33 (d, *J* = 5.7 Hz, 2H), 7.21-7.17 (m, 1H), 6.98-6.85 (m, 4H), 6.57-6.55 (m, 1H), 5.62 (m, 1H), 4.10 (m, 2H), 1.48 (m, 3H). |

### Example 6 Preparation of compound NRB156

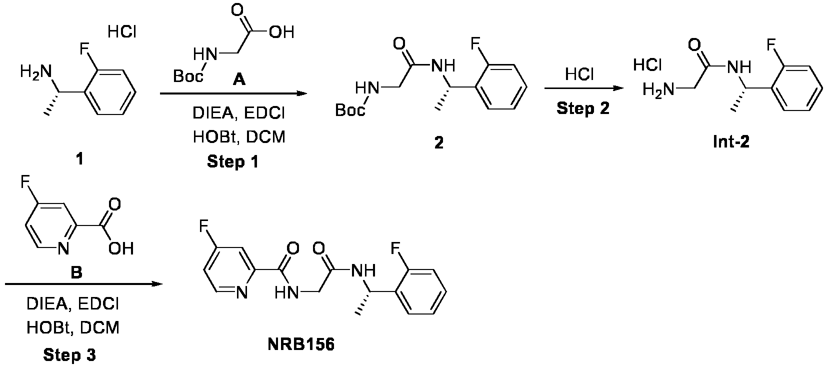

### Step 1:

Raw material 1 (1.5 g, 5.69 mmol) and raw material A (1.2 g, 6.86 mmol), EDCl (2.18 g, 11.39 mmol), HOBt (1.54 g, 11.39 mmol), and diisopropylethylamine (1.47 g, 11.393 mmol) were dissolved in 20 mL of dichloromethane. The mixture was stirred at room temperature for 2 hours. The completion of the reaction was monitored by TLC. A saturated aqueous sodium chloride solution (20 mL) was added, and the mixture was extracted with dichloromethane (3 × 10 mL). The organic phase was dried over anhydrous sodium sulfate, concentrated to dryness, and purified by column chromatography to obtain 1.6g of the product as a colorless oil. Analysis data: LCMS (ESI+): m/z 297 (M+H).

### Step 2:

Intermediate 2 (1.08 g, 3.68 mmol) was dissolved in methanol (5 mL) and HCl in ether solution (5 mL, concentration approximately 4 mol/L) was added. The reaction was stirred at room temperature for 1.5 hours. The completion of the reaction was monitored by TLC. After the reaction was completed, 746 mg of the product were obtained by vacuum concentration. Analysis data: LCMS (ESI+): m/z 197 (M+H).

### Step 3:

Intermediate Int-2 (40 mg, 0.17 mmol) and raw material B (29 mg, 0.206 mmol), EDCl (66 mg, 0.344 mmol), HOBt (46 mg, 0.344 mmol), and diisopropylethylamine (66 mg, 0.516 mmol) were dissolved in 1 mL of dichloromethane and stirred at room temperature for 1 hour. The completion of the reaction was monitored by TLC. The reaction was quenched by adding a saturated NaCl aqueous solution (5 mL) and then extracted with dichloromethane/methanol (10/1, 3 × 5 mL). The organic phase was dried over anhydrous sodium sulfate. After concentration, the crude product was purified by preparative thin-layer chromatography to obtain 39 mg product as a white solid. Analysis data: LCMS (ESI+): m/z 320 (M+H); ¹HNMR (300 MHz, DMSO-d6) δ 8.94-8.86 (m, 1H), 8.74-8.68 (m, 1H), 8.53 (d, J = 7.8 Hz, 1H), 7.82 (dd, J = 9.6, 2.1 Hz, 1H), 7.60-7.54 (m, 1H), 7.47-7.36 (m, 1H), 7.34-7.24 (m, 1H), 7.22-7.01 (m, 2H), 5.18 (q, J = 7.2 Hz, 1H), 4.04-3.98 (m, 2H), 1.36 (d, J = 7.2 Hz, 3H).

### Example 7 Preparation of compound NRB162-b

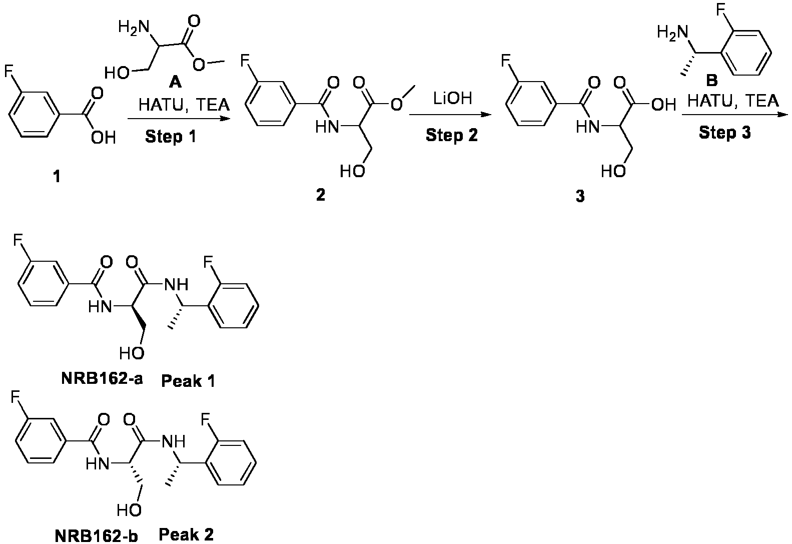

### Step 1:

Raw material 1 (300 mg, 2.14 mmol) and raw material A (365 mg, 2.36 mmol), HATU (1.22 g, 3.21 mmol), Et₃N (432 mg, 4.29 mmol) were dissolved in 20 mL of dichloromethane and stirred at room temperature for 2 hours. The completion of the reaction was monitored by TLC. A saturated aqueous sodium chloride solution (50 mL) was added, and the mixture was extracted with dichloromethane (3 × 10 mL). The organic phase was dried over anhydrous sodium sulfate, concentrated to dryness, and purified by column chromatography to obtain 335 mg of the product as a colorless oil. Analysis data: LCMS (ESI+): m/z 242 (M+H).

### Step 2:

Intermediate 2 (335 mg, 1.37 mmol) was dissolved in methanol (15 mL) and a solution of LiOH.H₂O (66 mg, 2.74 mmol) dissolved in 5 mL of water was added. Stirred at room temperature for 2 hours. The completion of the reaction was monitored by TLC. After the reaction, methanol was removed by vacuum concentration. The aqueous phase was then extracted with ethyl acetate to eliminate impurities. Subsequently, the aqueous phase was adjusted to pH 2 using 1M HCl. It was further extracted with DCM/MeOH (10:1, v/v) three times (3 × 30 mL). The organic phases were combined and dried, followed by vacuum concentration to obtain 175 mg of the product as a yellow oil. Analysis data: LCMS (ESI+): m/z 228 (M+H).

### Step 3:

Raw material 3 (100 mg, 0.44 mmol) and raw material B (93 mg, 0.53 mmol), HATU (251 mg, 0.66 mmol), Et₃N (89 mg, 0.88 mmol) were dissolved in 10 mL of dichloromethane and stirred at room temperature for 2 hours. The completion of the reaction was monitored by TLC. A saturated aqueous sodium chloride solution (20 mL) was added, and the mixture was extracted with dichloromethane (3 × 10 mL). The organic phase was dried over anhydrous sodium sulfate, concentrated to dryness, and purified by column chromatography to obtain 57 mg of product as a colorless oil. Through further separation, 17.8 mg of 162-a (peak 1) and 17.7 mg of 162-b (peak 2) were obtained. Analysis data: 162-a: LCMS (ESI+): m/z 349 (M+H); ¹HNMR (300 MHz, CD₃OD) 7.74-7.65 (m, 1H), 7.62-7.60 (m, 1H), 7.54-7.47 (m, 1H), 7.42-7.36 (m, 1H), 7.33-7.22 (m, 2H), 7.16-7.06 (m, 2H), 5.31-5.24 (m, 1H), 4.70-4.60 (m, 1H), 3.87-3.85 (m, 2H), 1.48 (d, J = 6.9 Hz, 3H).

**162-b:** LCMS (ESI+): m/z 349 (M+H); ¹HNMR (300 MHz, CD₃OD) □ 7.67-7.61 (m, 1H), 7.59-7.52 (m, 1H), 7.49-7.45 (m, 1H), 7.43-7.38 (m, 1H), 7.32-7.21 (m, 2H), 7.14-7.00 (m, 2H), 5.30-5.25 (m, 1H), 4.70-4.67 (m, 1H), 3.91-3.88 (m, 2H), 1.47 (d, J = 6.9 Hz, 3H).

### Example 8 Preparation of compound NRB181

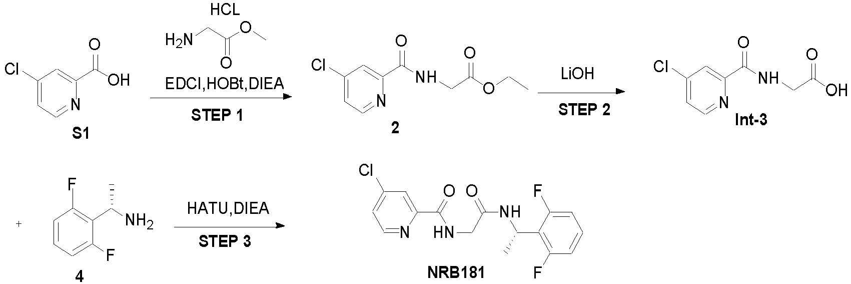

### Step 1:

S1 (2 g, 12.7 mmol) and glycine ethyl ester hydrochloride (1.75 g, 14.0 mmol), EDCl (3.16 g, 16.5 mmol), HOBt (2.51 g, 16.5 mmol), and diisopropylethylamine (4.9 g, 38.1 mmol) were dissolved in 60 mL of dichloromethane and stirred overnight at room temperature. The completion of the reaction was monitored by TLC. The reaction was quenched by adding an aqueous solution (100 mL) and then extracted with dichloromethane (3 × 80 mL). The organic phase was dried over anhydrous sodium sulfate, concentrated to dryness, and purified by column chromatography to obtain 478 mg of product as an off-white solid. Analysis data: LCMS (ESI+): m/z 229 (M+H).

### Step 2:

Intermediate 2 (478 mg, 2.1 mmol) was dissolved in tetrahydrofuran (6 mL) and water (3 mL), and lithium hydroxide (352 mg, 8.4 mmol) was added. The reaction was stirred at room temperature for 2 hours. The completion of the reaction was monitored by TLC. After the reaction, the reaction was concentrated to remove most of the tetrahydrofuran. The remaining reaction solution was then added with 50 mL of water and extracted with ethyl acetate to remove impurities. The remaining aqueous phase was adjusted to a pH of approximately 4, extracted three times with ethyl acetate (3 × 30 mL). The organic phase was dried with anhydrous sodium sulfate, and concentrated to obtain 325 mg of white solid Int-3. Analysis data: LCMS (ESI+): m/z 215 (M+H).

### Step 3:

Intermediate Int-3 (30 mg, 0.14 mmol) and (S) -1- (2,6-difluorophenyl) ethylamine (33 mg, 0.17 mmol), HATU (106 mg, 0.28 mmol), diisopropylethylamine (54 mg, 0.42 mmol) were dissolved in 1 mL of dichloromethane and stirred at room temperature for 3 hours. The completion of the reaction was monitored by TLC. The reaction was quenched by adding an ammonium chloride aqueous solution (5 mL), and the mixture was extracted with dichloromethane (3 × 5 mL). The organic phase was dried with anhydrous sodium sulfate, concentrate to dryness, and separated by preparative TLC plate to obtain 29 mg of white solid product. Analysis data: LCMS (ESI+): m/z 354 (M+H); ¹HNMR (300 MHz, CD₃OD) δ 8.57 (d, J = 5.1 Hz, 1H), 8.08 (s, 1H), 7.62 (d, J = 3.3 Hz, 1H), 7.32-7.22 (m, 1H), 6.92 (t, J = 8.1 Hz, 2H), 5.49-5.41 (m, 1H), 4.11-4.06 (m, 2H), 1.53 (d, J = 7.2 Hz, 3H).

### Example 9 Preparation of compound NRB211

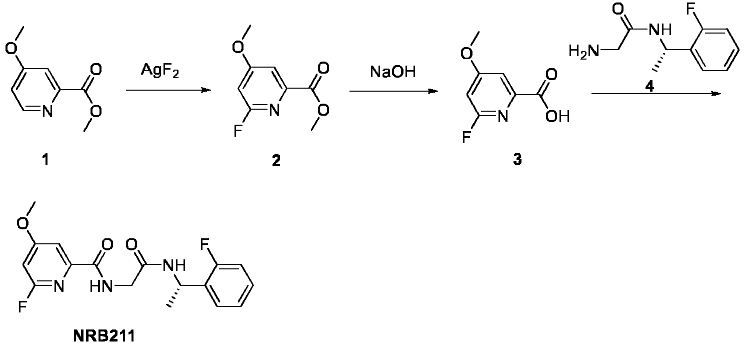

### Step 1:

1 (0.5 g, 3.0 mmol) was dissolved in anhydrous acetonitrile (10 mL), silver difluoride (1.3 g, 8.9 mmol) was added to the reaction under nitrogen protection, and reacted overnight at room temperature. The reaction was monitored by TLC to ensure that there is still a small amount of raw material remaining. A saturated sodium bicarbonate solution (30 mL) was added to adjust the pH to 10, extracted with ethyl acetate (2 × 30 mL), the organic layer was washed with water (2 × 20 mL), and then was washed with saturated sodium chloride solution (20 mL). The organic phase was dried with anhydrous sodium sulfate, filtered and concentrated, and purified by column chromatography to obtain 160 mg of the product as a yellow solid product. Analysis data: LCMS (ESI+): m/z 186 (M+H).

### Step 2:

**2** (160 mg, 0.86 mmol) was dissolved in methanol free solution (10 mL), and then 15% sodium hydroxide solution (3 mL) was added. Stirred at room temperature for 30 minutes and the completion of the reaction was monitored by TLC The reaction solution was concentrated until most of the methanol was evaporated, the pH was adjusted to 3 with 2N hydrochloric acid solution, extracted with ethyl acetate (2 × 5 mL), dry with anhydrous sodium sulfate, filtered and concentrated to obtain 140 mg of solid. Analysis data: LCMS (ESI -): m/z 170 (M-H).

### Step 3:

Raw material 3 (28.6 mg, 0.168 mmol), HOBt (41 mg, 0.30 mmol), EDCl (47.2 mg, 0.30 mmol) were dissolved in 2 mL of N, N-dimethylformamide and stirred for 10 minutes. Then raw material 4 (30 mg, 0.15 mmol) was added and stirred at room temperature for 1 hour. The completion of the reaction was monitored by TLC. The reaction was quenched by adding an aqueous solution (20 mL), and the mixture was extracted with ethyl acetate (3 × 10 mL). The organic phase was dried with anhydrous sodium sulfate, concentrated and purified on a preparative TLC plate to obtain 30 mg of the product. LCMS (ESI+): m/z 350 (M+H).¹H NMR (300 MHz, CD₃OD) δ 7.55 (s, 1H), 7.37 (t, J = 7.2 Hz, 1H), 7.28-7.22 (m, 1H), 7.18-7.11 (m, 1H), 7.10-7.00 (m, 1H), 6.77 (d, J = 1.5 Hz, 1H), 5.27 (q, J = 6.9 Hz, 1H), 4.14 (s, 2H), 3.95 (s, 3H), 1.46 (d, J = 6.9 Hz, 3H).

### Examples 10-12

The method of Example 16 was adopted, with the difference being that different substrates were used to replace intermediate 3, resulting in the preparation of compounds 17-20.

| NO. | Compound | Analysis data |
|---|---|---|
| 10 | 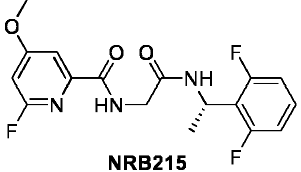 | LCMS (ESI+): m/z 368 (M+H).¹H NMR (300 MHz, CD₃OD) *δ* 7.54 (s, 1H), 7.32-7.22 (m, 1H), 6.92 (t, *J* = 8.4 Hz, 2H), 6.77 (d, *J* = 1.8 Hz, 1H), 5.48-5.41 (m, 1H), 4.12-4.00 (m, 2H), 3.95 (s, 3H), 1.53 (d, *J* = 6.9 Hz, 3H). |
| 11 | 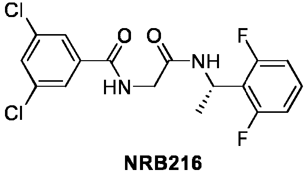 | LCMS (ESI+): m/z 387 (M+H); ¹HNMR (300 MHz, CD₃OD) *δ* 7.80 (d, *J* = 1.2 Hz, 2H), 7.75 (s, 1H), 7.33-7.22 (m, 1H), 6.93 (t, *J* = 8.4 Hz, 2H), 5.48-5.41 (m, 1H), 4.01 (q, *J=* 16.5 Hz, 2H), 1.54 (d, *J* = 7.2 Hz, 3H). |
| 12 | 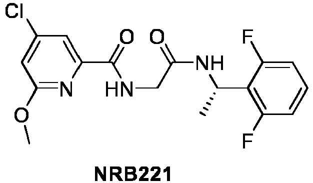 | LCMS (ESI+): m/z 384 (M+H); ¹HNMR (300 MHz, CDCl₃) *δ* 8.32 (brs, 1H), 7.81 (s, 1H), 7.21-7.15 (m, 1H), 6.93-6.84 (m, 3H), 6.63 (d, *J* = 5.7 Hz, 1H), 5.63 (q, *J* = 7.2 Hz, 1H), 4.12 (d, *J* = 5.4 Hz, 2H), 3.99 (s, 3H), 1.55 (d, *J* = 7.2 Hz, 3H). |

### Example 13 Preparation of compound NRB222

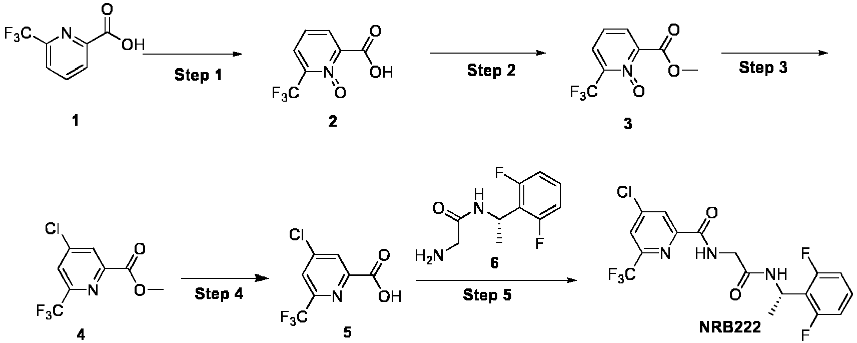

### Step 1:

Raw material 1 (2.0 **g**, 10.47 mmol) and 30% hydrogen peroxide (1.78 g, 15.70 mmol) was dissolved in trifluoroacetic acid (8 mL) and reacted at 80-90 °C for 8 hours. The reaction was monitored by TLC, revealing a small amount of residual starting material. The solvent was directly evaporated under reduced pressure to obtain 2.0 g of a yellow solid. Analysis data: LCMS (ESI+): m/z 208 (M+H).

### Step 2:

Raw material 2 (2.3 g, 11.11 mmol) was dissolved in hydrogen chloride/methanol (40 mL), stirred overnight at room temperature. And the completion of the reaction was monitored by TLC. The reaction solution was concentrated until most of the methanol was evaporated, the pH was adjusted to 7-8 with saturated sodium carbonate solution, and the mixture was extracted with methyl tert butyl ether (2 × 50 mL). The organic phase was washed with saturated sodium chloride (50 mL), dried with anhydrous sodium sulfate, filtered and concentrated to obtain 2.0 g of crude product. Analysis data: LCMS (ESI -): m/z 222 (M+H).

### Step 3:

Raw material 3 (2.0 g, 9.05 mmol) was dissolved in phosphorus oxychloride (10 mL) and stirred at 110 °C for 4 hours. The completion of the reaction was monitored by TLC. The pH was adjusted to 7-8 with saturated sodium carbonate solution, and the mixture was extracted with ethyl acetate (2 × 100 mL). The organic phase was washed with saturated sodium chloride (100 mL), dried with anhydrous sodium sulfate, concentrated to dryness, and purified by column chromatography to obtain 800 mg of yellow solid. LCMS (ESI+): m/z 240 (M+H).

### Step 4:

Raw material 4 (100 mg, 0.42 mmol) was dissolved in anhydrous methanol (4 mL), then sodium hydroxide (35 mg, 0.84 mmol) and water (2 mL) were added, and stirred at room temperature for 30 minutes. The completion of the reaction was monitored by TLC. The reaction solution was concentrated until most of the methanol was evaporated, the pH was adjusted to 3 with 2N hydrochloric acid solution, and the mixture was extracted with dichloromethane/methanol=10/1 (2 × 10 mL). The organic phase was dried with anhydrous sodium sulfate, filtered and concentrated to obtain 100 mg of yellow solid. Analysis data: LCMS (ESI -): m/z 224 (M-H).

### Step 5:

Raw material 5 (23 mg, 0.103 mmol), HOBt (26 mg, 0.186 mmol), EDCl (47.2 mg, 0.186 mmol) was dissolved in 2 mL of N, N-dimethylformamide and stirred for 10 minutes. Then raw material 6 (20 mg, 0.093 mmol) was added and stirred at room temperature for 2 hours. The completion of the reaction was monitored by TLC. The reaction was quenched by adding ice water (50 mL), and then extracted with ethyl acetate (2 × 25 mL). The organic phase was washed with water (50 mL) and saturated sodium chloride (50 mL). The organic phase was taken and dried with anhydrous sodium sulfate, concentrated to dryness, and purified on a preparative TLC plate to obtain 15 mg of white solid. LCMS (ESI+): m/z 422 (M+H); ¹H NMR (300 MHz, CD₃OD) δ 8.33 (s, 1H), 8.12 (s, 1H), 7.32-7.23 (m, 1H), 6.92 (t, J = 8.4 Hz, 2H), 5.44 (q, J = 7.2 Hz, 1H), 4.10 (q, J = 16.5 Hz, 2H), 1.53 (d, J = 7.2 Hz, 3H).

### Examples 14-15

The method of Example 13 was adopted, with the difference being that different substrates were used to replace intermediate 3, resulting in the preparation of compounds 17-20.

| NO. | Compound | Analysis data |
|---|---|---|
| 14 | 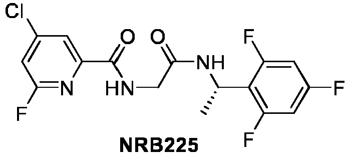 | LCMS (ESI+): m/z 390 (M+H); ¹H NMR (300 MHz, CD₃OD) *δ* 8.00 (s, 1H), 7.44 (s, 1H), 6.92 (t, *J* = 8.7 Hz, 2H), 5.45 (q, *J* = 7.2 Hz, 1H), 4.11 (q, *J* = 16.5 Hz, 2H), 1.52 (d, *J* = 7.2 Hz, 3H) |
| 15 | 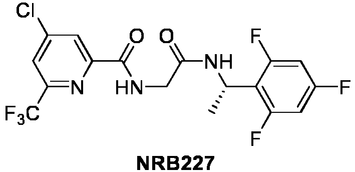 | LCMS (ESI+): m/z 440 (M+H); ¹H NMR (300 MHz, CD₃OD) *δ* 8.33 (s, 1H), 8.13 (s, 1H), 6.82 (t, *J* = 8.7 Hz, 2H), 5.36 (q, *J* = 7.2 Hz, 1H), 4.14 (q, *J* = 16.5 Hz, 2H), 1.53 (d, *J* = 7.2 Hz, 3H). |

### Example 16

### Preparation of compound NRB214

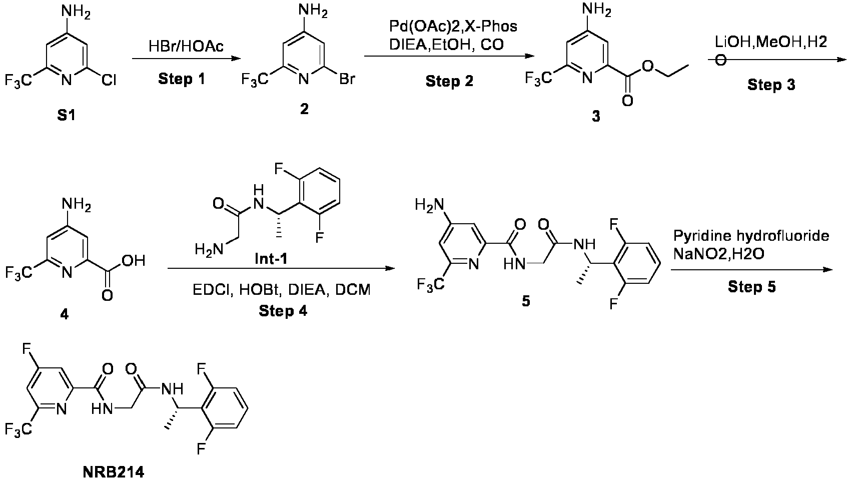

### Step 1:

1 g of S1 was dissolved in 10 mL of HBr acetic acid solution, heated to 110 °C and stirred overnight. The raw material was monitored by HPLC and found to remain 40%. The reaction solution was concentrated and then dissolved in 10 mL of HBr acetic acid solution. The mixture was heated to 110°C and stirred overnight. The raw material was monitored by HPLC and found to remain about 13%. The reaction solution was concentrated, NaHCO₃ aqueous solution (20 mL) was added to quench the reaction, extracted with ethyl acetate (2 × 20 mL). The organic phase was dried with anhydrous sodium sulfate, and concentrated to obtain 1.5 g of crude yellow solid. Analysis data: LCMS (ESI+): m/z 241,243 (M+H).

### Step 2:

Intermediate 2 (1.5 g, 6.2 mmol) was dissolved in ethanol (20 mL), palladium acetate (140 mg, 0.62 mmol), X-Phos (593 mg, 1.2 mmol), and diisopropylethylamine (803 mg, 6.2 mmol) were added. Heated to 80°C and stirred overnight in CO system. The raw material was monitored by HPLC and found to remain 70%. After filtering the reaction solution, palladium acetate (140 mg, 0.62 mmol), X-Phos (593 mg, 1.2 mmol), and diisopropylethylamine (803 mg, 6.2 mmol) were added again. Heated to 80°C and stirred overnight in CO system. The raw material was monitored by HPLC and found to remain about 15%. The reaction solution was filtered and concentrated, then separated and purified by column chromatography to obtain 630 mg of yellow oily product. Analysis data: LCMS (ESI+): m/z 235 (M+H).

### Step 3:

Intermediate 3 (630 mg, 2.7 mmol) and LiOH.H₂O (230 mg, 5.4 mmol) was dissolved in 10 mL of methanol and 5 mL of water, and stirred at room temperature for 1 hour. The completion of the reaction was monitored by LCMS. The reaction solution was directly concentrated by adding 5 mL of water and washed with ethyl acetate (2 × 5 mL). The aqueous phase was adjusted to pH 5-6 with 1 mol of dilute hydrochloric acid, and then concentrated directly to obtain 500 mg of crude yellow solid product. Analysis data: LCMS (ESI+): m/z 207 (M+H).

### Step 4:

Intermediate 4 (330 mg, 1.6 mmol) and Int-1 (274 mg, 1.3 mmol), EDCl (615 mg, 3.2 mmol), HOBt (432.5 mg, 3.2 mmol), and diisopropylethylamine (413.3 mg, 3.2 mmol) were dissolved in 10 mL of dichloromethane and stirred at room temperature for 6 hours. The completion of the reaction was monitored by TLC. The reaction was quenched by adding an ammonium chloride aqueous solution (10 mL), extracted with dichloromethane (2 × 10 mL). The organic phase was dried with anhydrous sodium sulfate, concentrated to dryness, and purified by column chromatography to obtain 120 mg of white solid product. Analysis data: LCMS (ESI+): m/z 403 (M+H).

### Step 5:

Intermediate 5 (50 mg, 0.12 mmol) was dissolved in 1 mL of pyridine hydrogen fluoride and stirred at 0 °C for 10 minutes in a tetrafluoroethylene bottle. 0.5 mL of an aqueous solution of nitrous acid (12.8 mg, 0.19 mmol) was added dropwise Stirred overnight at 0°C in a PTFE bottle. The completion of the reaction was monitored by LCMS. The reaction was quenched by adding triethylamine (2 mL), extracted with ethyl acetate (2 × 2 mL). The organic phase was dried with anhydrous sodium sulfate, concentrated to dryness, and purified by preparative TLC to obtain 7.6 mg of white solid product. Analysis data: LCMS (ESI+): m/z 406 (M+H); ¹H-NMR (300 MHz, CDCl₃) δ 8.40-8.34 (m, 1H), 8.10 (d, J = 8.4 Hz, 1H), 7.56 (dd, J = 7.2, 2.1 Hz, 1H), 7.21-7.15 (m, 1H), 6.90-6.84 (m, 2H), 6.57-6.53 (m, 1H), 5.64 (q, J = 7.5 Hz, 1H), 4.21-4.07 (m, 2H), 1.53 (d, J = 7.5 Hz, 3H).

### Example 17 Determination of GPR139 receptor activity of the compound of the present invention

### 1. Experimental methods

HEK293 cells were grown in a 10 cm culture dish containing DMEM complete medium (containing glutamine, sodium pyruvate, penicillin/streptomycin, 10% fetal bovine serum) and incubated at 37 °C in a 5% CO₂ incubator until 90% confluency. Then subculture twice a week at a ratio of 1:5. During cell passaging, the culture medium was first completely removed, washed once with 4 mL of DPBS, and then 3 mL of trypsin digestion solution was added and mixed well. When cell dissociation was observed, the trypsin solution was aspirated and 5 mL of complete DMEM medium was added. The cells were dispersed by pipetting, and 1 mL of cell suspension was added to each of five new 10 cm culture dishes. Then, 7 mL of complete DMEM medium was supplemented to each dish. After mixing, the cells were cultured at 37°C in a 5% CO₂ incubator.

On the transfection day, cells were digested and seeded into one 10 cm culture dish at appropriate density. 6 mL of DMEM/F 12 medium (containing glutamine, sodium pyruvate, HEPES, and fetal bovine serum) was added, and the cells were cultured for 5 hours at 37°C in 5% CO₂. A 50 mL sterile centrifuge tube (Tube A) was taken, and 1 mL of serum-free DMEM/F12 medium (containing glutamine, sodium pyruvate, and HEPES) was added, followed by 10 µg of GPR139 DNA. The mixture was mixed well. Another 50 mL sterile centrifuge tube (Tube B) was taken, and 1 mL of serum-free DMEM medium (containing glutamine, sodium pyruvate, and HEPES) was added, followed by 60 µL of Fugene HD transfection reagent. The mixture was mixed well. 1 mL of Fugene HD transfection reagent from Tube B was transferred to Tube A, mixed well, and incubated at room temperature for 20 minutes. 2 mL of DMEM/F12 medium (containing glutamine, sodium pyruvate, HEPES, and fetal bovine serum) was added to Tube A. After mixing, 4 mL of mixture from Tube A was transferred to the prepared 10 cm dish (total volume 10 mL). The cells were cultured for 20-24 hours at 37°C in 5% CO₂.

The next day, the transfected cells were digested with trypsin digestion solution, and appropriate DMEM/F12 medium (containing glutamine, sodium pyruvate, HEPES, penicillin/streptomycin, fetal bovine serum) was added. The cells were seeded into Matrigel coated 384-well plates with a cell density of 6.67 × 10⁶ cells/mL, 30 µ L/well. The 384-well plates were cultured at 37°C in 5% CO₂.

On the third day, 10 µL/well of FLIPR dye (diluted in HEPES solution containing Pluronic and Probenecid) was added to the 384-well cell plate. And the plate was incubated for 40 minutes at 37°C in 5% CO₂. Diluted compounds were added to the cell plate and calcium signals were measured using a FLIPR plate reader.

Data were analyzed using XL-fit software to obtain EC50 values for each compound.

### 2. Experimental results

**Table 1: EC50 values of compounds for GPR139 FLIPR cell calcium signal detection**

| Compound | GPR139 | Compound | GPR139 |
|---|---|---|---|
| NRB153 | *** | NRB154 | ** |
| NRB156 | ** | NRB162-b | ** |
| NRB178 | *** | NRB180 | *** |
| NRB181 | *** | NRB202 | *** |
| NRB211 | *** | NRB214 | ** |
| NRB215 | *** | NRB216 | *** |
| NRB221 | ** | NRB222 | ** |
| NRB225 | *** | NRB227 | ** |
| *1-10 µM; ** 0.1-1 µM; *** 0.1-100 nM | | | |

### Example 18 The pharmacological effect of compound NRB181 in acute restraint mouse stress model

### 18.1 Experimental Materials

### 18.1.1 Experimental animals

Mouse: Male C57BL/6J; 8 weeks old; Weight: 20-25g; Quantity: 40 pieces
Feeding requirements: 5 mice per cage; the animal room alternates between light and dark for 12 hours; no restrictions on food and water intake; before the experiment

Animals were purchased to adapt to the environment within one week.

### 18.1.2 Corticosterone detection kit

Cloud-clone Corp.wuhan CEA540Ge

### 18.2 Experimental groups

Group 1, acute restraint + placebo group, 10 mice; the mice were divided into 2 cages, 5 mice per cage. On the morning of the day of the experiment, the mice in this group were subjected to acute restraint, during which they were fasted and deprived of water.

Group 2, acute restraint + low-dose of compound NRB181 group (1mg/kg), 10 mice; the mice were divided into 2 cages, 5 mice per cage. On the morning of the day of the experiment, the mice in this group were subjected to acute restraint, during which they were fasted and deprived of water.

Group 3, acute restraint + moderate-dose of compound NRB181 group (5mg/kg), 10 mice; the mice were divided into 2 cages, 5 mice per cage. On the morning of the day of the experiment, the mice in this group were subjected to acute restraint, during which they were fasted and deprived of water.

Group 4, acute restraint + high-dose of compound NRB181 group (25mg/kg), 10 mice; the mice were divided into 2 cages, 5 mice per cage. On the morning of the day of the experiment, the mice in this group were subjected to acute restraint, during which they were fasted and deprived of water.

### 18.3 Procedures of Animal Experiment

18.3.1 On the day before the experiment, a placebo was prepared: 1 mL of 100% DMSO and 9 mL of formula x solution was added. Preparation of formula x: First, saturated β - cyclodextrin was prepared, that was, 0.2g of cyclodextrin was dissolved in 10 mL of saline, and 4 mL of saturated cyclodextrin was added to 16 mL of saline to obtain formula x.

### 18.3.2 Preparation of compound NRB181 solution on the day before the experiment:

The preparation of high-dose NRB181 solution was as follows: 7.5 mg of NRB181 was weighed, and dissolved in 300ul of 100% DMSO, mix well, 2.7 mL of formula x solution was added, sonicated for 10 minutes, and a 2.5 mg/mL solution (high-dose) was prepared.

The preparation of the moderate-dose NRB181 solution was as follows: 0.6 mL of the above high-dose solution was taken and added to 2.4 mL of placebo, sonicated for 10 minutes, and a 0.5mg/mL solution (moderate-dose) was prepared.

The preparation of low-dose NRB181 solution was as follows: 0.5 mL of the above moderate-dose solution was taken and added to 2 mL of placebo, sonicated for 10 minutes, and a 0.1mg/mL solution (low-dose) was prepared. The above three solutions should be stored at 4 degrees Celsius for later use, and sonicated for 10 minutes before the experiment.

18.2.3 On the morning of Day 1 of the experiment, the experimental mice in Groups 1-4 were placed in the behavioral laboratory and allowed to adapt for 1 hour.

18.2.4 Mice of Group 1 were administered a placebo by gavage, 0.1 mL/10g. After 30 minutes, 10 mice were sequentially placed into 10 bound centrifuge tubes, with their tails leaking out through the centrifuge tube lid. Acute dynamic restraint was performed, during which they were fasted and deprived of water.

18.2.5 Mice of Group 2 were administered a low-dose NRB181 solution by gavage, 0.1 mL/10g. After 30 minutes, 10 mice were sequentially placed into 10 bound centrifuge tubes, with their tails leaking out through the centrifuge tube lid. During which they were fasted and deprived of water.

18.2.6 Next, a moderate-dose of NRB181 was administered to the Group 3 and a high-dose of NRB181 was administered to the Group 4 by gavage, and acute restraint was performed on each mouse as in the previous steps.

After acute restraint for 3 hours from Group 1 to Group 4, mice were euthanized and their blood was collected.

18.2.7 Corticosterone level testing was performed on the above 40 samples first.

18.4 ELISA detection of corticosterone content in plasma: the dilution factor of the plasma sample was 20 times, and the specific detection method should refer to the instructions of the ELISA kit (CEA540Ge).

### 18.5 Experimental Results

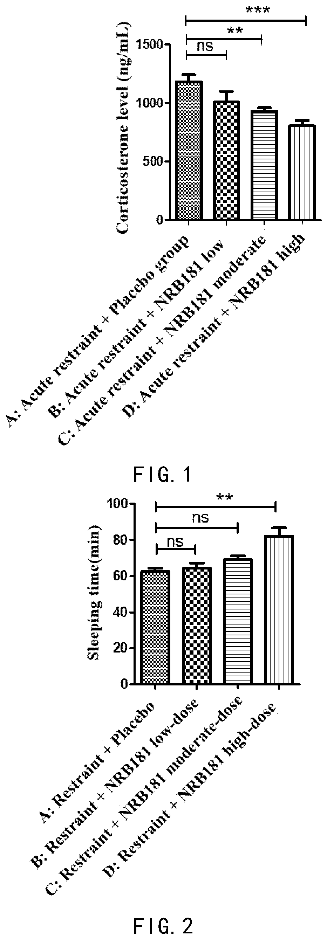

The experimental results were shown in FIG.1. Compound NRB181 significantly reduced the expression level of corticosterone in mice after 30 minutes of administration and 3 hours of acute restraint, showing a dose-dependent relationship.

### Example 19 Effect of compound NRB181 on sleep in mice under acute restraint stress

### 19.1 Experimental animals

Mouse: Male C57BL/6J; 8 weeks old; Weight: 20-25g; Quantity: 31 pieces
Feeding requirements: 7 or 8 mice per cage; the animal room alternates between light and dark for 12 hours; no restrictions on food and water intake; animals were purchased to adapt to the environment one week before the experiment.

### 19.2 Experimental groups

Group 1, acute restraint sleeping placebo group, 7 mice, fed with regular care.

Group 2, acute restraint sleeping + low-dose of compound NRB181 group, 8 mice. The mice were fasted and deprived of water during the acute restraint period, and were kept normally at other times.

Group 3, acute restraint sleeping + moderate-dose of compound NRB181 group, 8 mice. The mice were fasted and deprived of water during the acute restraint period, and were kept normally at other times.

Group 4, acute restraint sleeping + high-dose of compound NRB181 group, 8 mice. The mice were fasted and deprived of water during the acute restraint period, and were kept normally at other times.

### 19.3 Procedures of Animal Experiment

19.3.1 Preparation of placebo one day before the experiment: 0.5% CMC sodium + 1% Tween80 + saline.

On the day before the experiment, a high-dose solution of compound NRB181 was prepared: 6 mg of NRB181 was weighed and dissolved in 2.4 mL of placebo to prepare a 2.5 mg/mL solution of NRB181. Sonicated for 10 minutes and stored at 4°C for later use.

The day before the experiment, a moderate-dose solution of compound NRB181 was prepared: 450uL of the above high-dose solution was taken and added to a 2 mL placebo centrifuge tube, mixed well, a 0.5 mg/mL NRB181 solution was prepared, sonicated for 10 minutes, and stored at 4 ° C for later use.

The day before the experiment, a low-dose solution of compound NRB181 was prepared: 400ul of the above moderate-dose solution was taken and added to a 1.6 mL placebo centrifuge tube, mixed well, a 0.1 mg/mL NRB181 solution was prepared, sonicated for 10 minutes, and stored at 4 ° C for later use.

19.3.2 On the day of the experiment, the mice were placed in the behavioral laboratory and allowed to adapt for 1 hour.

19.3.3 Mice in Group 1 were administered a placebo by gavage at a dose of 0.1 mL/10g. After 30 minutes, 7 mice were sequentially placed into 7 bound centrifuge tubes, with their tails leaking out through the centrifuge tube lid. Acute restraint was performed for 3 hours, during which fasting and water deprivation are required. A sedative sleep test was conducted immediately on mice after the acute restraint was completed.

19.3.4 Mice of Group 2 were administered a low-dose solution of NRB181 by gavage, 0.1 mL/10g. After 30 minutes, 8 mice were sequentially placed into 8 bound centrifuge tubes, with their tails leaking out through the centrifuge tube lid. Acute restraint was performed for 3 hours, during which fasting and water deprivation are required. A sedative sleep test was conducted immediately on mice after the acute restraint was completed.

19.3.5 Mice of Group 3 were administered a moderate-dose solution of compound NRB181 by gavage, 0.1 mL/10g. After 30 minutes, 8 mice were sequentially placed into 8 bound centrifuge tubes, with their tails leaking out through the centrifuge tube lid. Acute restraint was performed for 3 hours, during which fasting and water deprivation are required. A sedative sleep test was conducted immediately on mice after the acute restraint was completed.

19.3.6 Mice of Group 4 was administered a high-dose solution of NRB181 by gavage, 0.1 mL/10g. After 30 minutes, 8 mice were sequentially placed into 8 bound centrifuge tubes, with their tails leaking out through the centrifuge tube lid. Acute restraint was performed for 3 hours, during which fasting and water deprivation are required. A sedative sleep test was conducted immediately on mice after the acute restraint was completed.

19.3.7 Upon completion of acute restraint of the first mice in Group 1, pentobarbital sodium (40 mg/kg) was administered immediately by intraperitoneal injection. Continue intraperitoneal injection of pentobarbital sodium (40mg/kg) in each mouse until the last mouse in Group 4 has been injected with pentobarbital sodium.

19.3.9 The sleep latency (disappearance time of righting reflex) and sleep time (time from disappearance to recovery of righting reflex) of mice in each group was observed after administration of pentobarbital sodium.

### 19.4 Experimental Results

The experimental results were shown in FIG.2. Compound NRB181 significantly increased the sleep time of pentobarbital sodium anesthetized mice after acute restraint for 3 hours 30 minutes after administration, and showed a dose-dependent relationship. In addition, to determine whether compound NRB181 has an effect on the metabolic enzymes of pentobarbital sodium, we used LC-MS method to detect the pentobarbital sodium content in mice anesthetized with pentobarbital sodium under placebo and compound action in the pilot experiment. The results showed that compound NRB181 had no effect on the metabolic enzymes of pentobarbital sodium.

### Example 20 The efficacy of compound NRB181 in Forced Swim Test

### 20.1 Experimental animals

Mouse strain: Male Crl: CD1 (ICR) mice; Weight: 35-40g; Quantity: 16 pieces
Feeding requirements: 4 per cage; The animal room alternates between light and dark for 12 hours; No restrictions on food and water.

### 20.2 Experimental groups

Group 1, placebo group: 8 mice, 4 mice per cage;
Group 2, NRB181 group: 8 mice, 4 mice per cage.

### 20.3 Procedures of Animal Experiment

20.3.1 Preparation of placebo before experiment: 10% DMSO + saline.
20.3.2 Preparation of compound NRB181 before experiment: 10mg of NRB181 was weighed and added to 4 mL of placebo, mixed well.
20.3.3 The Forced Swim Test will be completed from 14:00 to 17:00 in the afternoon.
20.3.4 On the morning of the experimental day, all mice were weighed and then evenly divided into each group based on their weight, with 8 mice per group, for a total of 16 mice. Then, single cage feeding should be carried out with the same bedding as in cage feeding, and food and water should be avoided;
20.3.5 The mice were transferred to the behavioral laboratory and allowed to adapt for 3 hours.
20.3.6 Drug injection: mice of Group 1 received intraperitoneal injection of placebo at a dose of 0.1 mL/10g; after Forced Swim Test of the mice in Group 1, mice in Group 2 were intraperitoneally injected with compound NRB181 at a dose of 0.1 mL/10g.
20.3.7 Forced Swim Test:
   Mice of Group 1 underwent Forced Swim Test in sequence 10 minutes after injection. The mice were placed in a glass jar (diameter 10cm, height 25 cm; water depth 15 cm, water temperature 23 °C) and swam for 6 minutes. The first 2 minutes were the adaptation period, and the last 4 minutes were the experimental period; The software records the immobility time, swimming time, and climbing time of mice within 4 minutes. After testing each mouse, the glass jar was rinsed with experimental water, new water (15 cm depth, 23 °C temperature) was added, and then the next mouse was tested; this process was repeated until all mice had been tested.

Mice of Group 2 underwent Forced Swim Test 10 minutes after injection of the drug, the same method as above was used.

### 20.4 Experimental Results

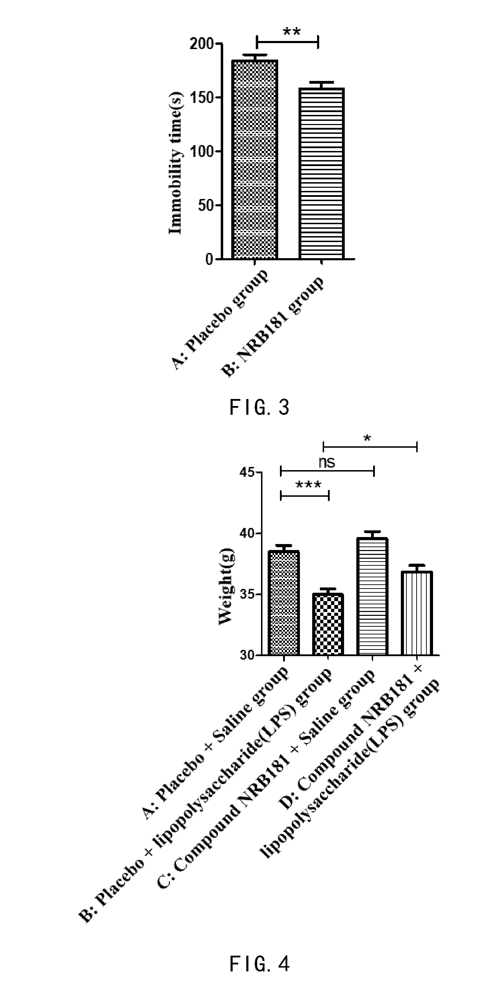

The experimental results were shown in FIG.3. Compound NRB181 can significantly reduce the time of despair in mice during Forced Swim Test.

### Example 21 Efficacy of compound NRB181 in lipopolysaccharide induced mouse depression model

### 21.1 Experimental animals

Mice: Male Crl: CD1 (ICR) mice; 10-14 weeks old; Weight: 35-40g; Quantity: 40 pieces
Feeding requirements: 5 mice per cage; the animal room alternates between light and dark for 12 hours; no restrictions on food and water intake; animals were purchased to adapt to the environment one week before the experiment.

### 21.2 Experimental groups

Group 1, placebo + saline group: 10 mcie; divided into 2 cages, 5 mice per cage.

Group 2, placebo + lipopolysaccharide (LPS) group: 10 mice; divided into 2 cages, 5 mice per cage.

Group 3, compound NRB181 + saline group: 10 mice; divided into 2 cages, 5 mice per cage.

Group 4, compound NRB181 + lipopolysaccharide (LPS) group: 10 mice; divided into 2 cages, 5 mice per cage.

### 21.3 Procedures of Animal Experiment

21.3.1 Preparation of placebo before experiment: 0.5% CMC sodium + 1% Tween80 + saline. In a clean bench, 4 tubes of 40 mL placebo were prepared using pyrogen-free centrifuge tubes and ultrapure water, totaling 160 mL, ensuring the absence of endotoxins (LPS). Sterile and endotoxin free centrifuge tubes were used to divide 3 tubes of placebo into 4 mL/tube, for a total of 30 tubes, and stored at 4 °C for future use.

### 21.3.2 Preparation of LPS sterile solution before experiment:

LPS with a purity of >98% was required 3.2mg of LPS was accurately weighed and dissolved in 40 mL of placebo to prepare a sterile solution of 0.08 mg/mL. After thorough mixing, the solution was divided into sterile and endotoxin-free centrifuge tubes, 4 mL per tube. The LPS solution needed for the next day was stored at 4 °C (3 tubes), while the rest was frozen at -20°C for future use.

### 21.3.3 Preparation of compound NRB181 solution before experiment:

87.5 mg of compound NRB181 was accurately weighed and dissolved in 35 mL of the aforementioned placebo to prepare a 2.5 mg/mL solution, which was then divided into 5 mL/tube. Sonicated for 10 minutes and stored at 4 °C.

21.3.4 On the first day of the experiment at 8:00 am, four groups of mice were randomly divided into groups, and the weight of each mouse was recorded before being placed back in their respective cages.

### 21.3.5 Experiment Day 1 at 9:00 am:

Mice in Group 1 were administered a placebo (0.1 mL/10 g) by gavage and were returned to their home cages.

Mice in Group 2 were administered a placebo (0.1 mL/10g) by gavage and were returned to their home cages.

Mice in Group 3 were administered a NRB181 solution (0.1 mL/10g) by gavage and were returned to their home cages.

Mice in Group 4 were administered a NRB181 solution (0.1 mL/10g) by gavage and were returned to their home cages.

### 21.3.6 Experiment Day 1 at 9:00 pm:

Mice in Group 1 were administered a placebo (0.1 mL/10g) by gavage and were returned to their home cages.

Mice in Group 2 were administered a placebo (0.1 mL/10g) by gavage and were returned to their home cages.

Mice in Group 3 were administered a NRB181 solution (0.1 mL/10g) by gavage and were returned to their home cages.

Mice in Group 4 were administered a NRB181 solution (0.1 mL/10g) by gavage and were returned to their home cages.

### 21.3.7 Experiment Day 2 at 9:00 am:

Mice in Group 1 were administered a placebo (0.1 mL/10g) by gavage and were returned to their home cages.

Mice in Group 2 were administered a placebo (0.1 mL/10g) by gavage and were returned to their home cages.

Mice in Group 3 were administered a NRB181 solution (0.1 mL/10g) by gavage and were returned to their home cages.

Mice in Group 4 were administered a NRB181 solution (0.1 mL/10g) by gavage and were returned to their home cages.

### 21.3.8 Experiment Day 2 at 9:00 pm:

Mice in Group 1 were administered a placebo (0.1 mL/10g) by gavage and were returned to their home cages.

Mice in Group 2 were administered a placebo (0.1 mL/10g) by gavage and were returned to their home cages.

Mice in Group 3 were administered a NRB181 solution (0.1 mL/10g) by gavage and were returned to their home cages.

Mice in Group 4 were administered a NRB181 solution (0.1 mL/10g) by gavage and were returned to their home cages.

### 21.3.9 Experiment Day 3 at 9:00 am:

Mice in Group 1 were administered a placebo (0.1 mL/10g) by gavage and were returned to their home cages.

Mice in Group 2 were administered a placebo (0.1 mL/10g) by gavage and were returned to their home cages.

Mice in Group 3 were administered a NRB181 solution (0.1 mL/10g) by gavage and were returned to their home cages.

Mice in Group 4 were administered a NRB181 solution (0.1 mL/10g) by gavage and were returned to their home cages.

### 21.3.10 Experiment Day 3 at 9:00 pm:

Mice in Group 1 were administered a placebo (0.1 mL/10g) by gavage and were returned to their home cages.

Mice in Group 2 were administered a placebo (0.1 mL/10g) by gavage and were returned to their home cages.

Mice in Group 3 were administered a NRB181 solution (0.1 mL/10g) by gavage and were returned to their home cages.

Mice in Group 4 were administered a NRB181 solution (0.1 mL/10g) by gavage and were returned to their home cages.

### 21.3.11 Experiment Day 4 at 9:00 am:

Mice in Group 1 were administered a placebo (0.1 mL/10g) by gavage and were returned to their home cages.

Mice in Group 2 were administered a placebo (0.1 mL/10g) by gavage and were returned to their home cages.

Mice in Group 3 were administered a NRB181 solution (0.1 mL/10g) by gavage and were returned to their home cages.

Mice in Group 4 were administered a NRB181 solution (0.1 mL/10g) by gavage and were returned to their home cages.

### 21.3.12 On the morning of Day 4 of the experiment, after the Group 4 was administered by gavage, immediately:

Mice in Group 1 were injected intraperitoneally with a saline solution (0.1 mL/10g) and were returned to their home cages.

Mice in Group 2 were injected intraperitoneally with a LPS solution (0.1 mL/10g) and were returned to their home cages.

Mice in Group 3 were injected intraperitoneally with a saline solution (0.1 mL/10g) and were returned to their home cages.

Mice in Group 4 were injected intraperitoneally with a LPS solution (0.1 mL/10g) and were returned to their home cages.
21.3.13 On the afternoon of the Day 4 of the experiment, 6 hours after mice in Group 1 were injected with saline, an Open Field Test was conducted on each group starting from Group 1. Relevant parameters were recorded, and then the mice in each group were returned to their respective home cages.
21.3.14 On the Day 5 of the experiment at 9:00 am, each group of mice was weighed and recorded, and then were returned to their home cages.
21.3.15 On the Day 5 of experiment, 24 hours after mice of Group 1 were injected with saline the day before, a Forced Swim Test was conducted on each group of mice starting from Group 1, and the immobility time of each mouse was recorded.
21.3.16 On the Day 5 of the experiment, after the Forced Swim Test was completed, each group of mice was euthanized and blood was collected.
21.3.17 Corticosterone level testing was performed on the above 40 samples first.

### 21.4 Experimental results

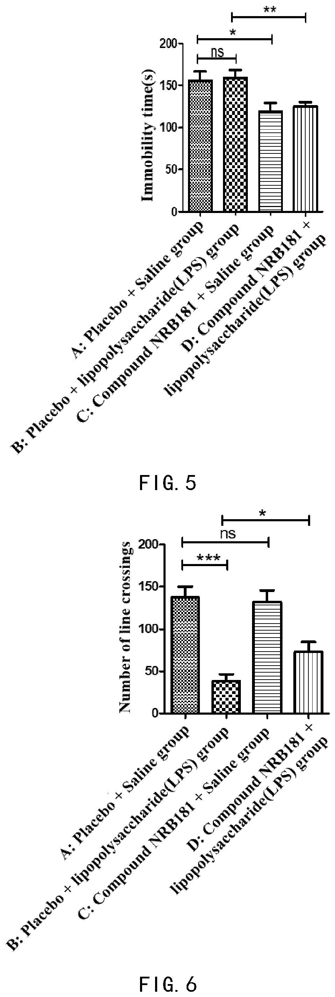

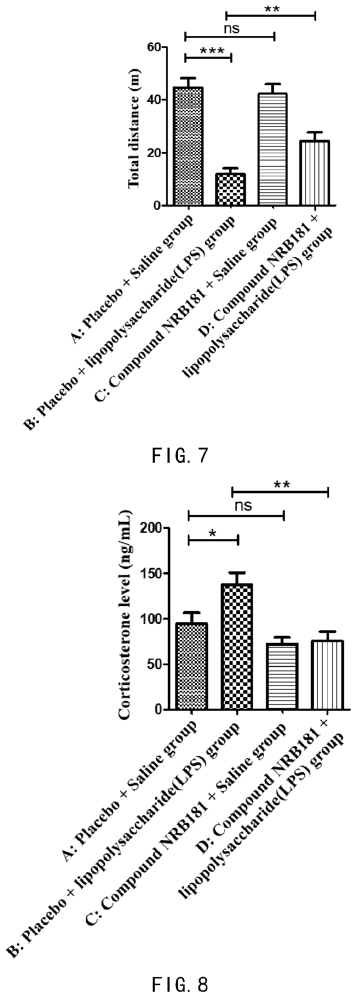

As shown in FIG.4, compound NRB181 can significantly alleviate weight loss in LPS induced mouse depression model; as shown in FIG.5, compound NRB181 can significantly reduce the despair time of mice in the Forced Swim Test in the LPS induced mouse depression model; as shown in FIG.6 and 7, compound NRB181 significantly increased the activity of mice in the Open Field Test in LPS induced mouse depression model; as shown in FIG.8, compound NRB181 can significantly reduce the expression level of corticosterone in LPS induced mouse depression model.

All references mentioned in the present invention are cited as references in this application, as if each reference were cited separately. In addition, it should be understood that after reading the above teaching content of the present invention, those skilled in the art can make various changes or modifications to the present invention, and these equivalent forms also fall within the scope of the claims attached to this application.

## Claims

1. A compound as shown in formula (I), a deuterated compound thereof, or a pharmaceutically acceptable salt thereof, 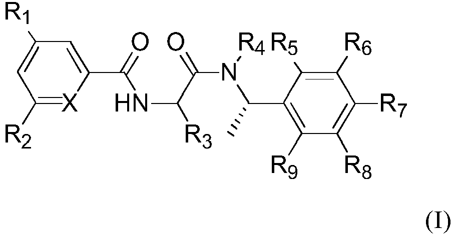 wherein,
X is selected from the group consisting of: N, CH;
R₁ and R₂ are each independently selected from the group consisting of: hydrogen, halogen, hydroxyl, carboxyl, C₁₋₃ alkyl, C₁₋₆ acyl, C₁₋₃ alkyl-carbonyl-C₁₋₃ alkyl, C₁₋₆ alkyl-hydroxyl, C₁₋₆ alkyl-aldehyde, C₁₋₆ alkyl, C₁₋₆ haloalkyl, C₁₋₆ alkoxy, and C₁₋₆ haloalkoxy;
R₃ is selected from the group consisting of: hydrogen, halogen, hydroxyl, carboxyl, C₁₋₆ acyl, C₁₋₃ alkyl-carbonyl-C₁₋₃ alkyl, C₁₋₆ alkyl-hydroxyl, C₁₋₆ alkyl-aldehyde, C₁₋₆ alkyl, C₁₋₆ haloalkyl, C₁₋₆ alkoxy, C₁₋₆ haloalkoxy, C₃₋₈ saturated or unsaturated carbocyclyl, 3-10 membered saturated or unsaturated heterocyclyl, C6-10 aryl, 5-12 membered heteroaryl;
R₄ is selected from the group consisting of: hydrogen, halogen, carboxyl, C₁₋₆ acyl, C₁₋₃ alkyl-carbonyl-C₁₋₃ alkyl, C₁₋₆ alkyl-hydroxyl, C₁₋₆ alkyl-aldehyde, C₁₋₆ alkyl, C₁₋₆ haloalkyl;
R₅, _{R6,} R₇, R₈, and _{R9} are each independently _{selected} from the _{group consisting of:} hydrogen, halogen, hydroxyl, _{carboxyl,} C₁₋₆ acyl, C₁₋₃ alkyl-carbonyl-C₁₋₃ alkyl, C₁₋₆ alkyl-aldehyde, C₁₋₆ alkyl, C₁₋₆ haloalkyl, C₁₋₆ alkoxy, C₁₋₆ haloalkoxy;
wherein, the carbocyclyl, heterocyclyl, aryl, or heteroaryl groups may be monocyclic, spirocyclic, fused-ring, or bridged-ring structural groups.

2. The compound of claim 1, a deuterated compound thereof, or a pharmaceutically acceptable salt thereof, wherein, R₁ and R₂ are each independently selected from the group consisting of: hydrogen, halogen, hydroxyl, carboxyl, C₁₋₃ alkyl, C₁₋₃ alkoxy, C₁₋₃ haloalkyl, and C₁₋₃ haloalkoxy.
In another preferred embodiment, R₁ and R₂ are each independently selected from the group consisting of: hydrogen, chlorine, fluorine, -CH₃, -CF₃, -OCH_{3∘}

3. The compound of claim 1, a deuterated compound thereof, or a pharmaceutically acceptable salt thereof, wherein, R₃ is selected from the group consisting of: hydrogen, halogen, hydroxyl, carboxyl, C₁₋₃ acyl, C₁₋₃ alkyl-carbonyl-C₁₋₃ alkyl, C₁₋₃ alkyl-hydroxyl, C₁₋₃ alkyl-aldehyde, C₁₋₃ alkyl, C₁₋₃ haloalkyl, and C₁₋₃ alkoxy.
In another preferred embodiment, R₃ is selected from the group consisting of: hydrogen, -CH₂OH_{∘}

4. The compound of claim 1, a deuterated compound thereof, or a pharmaceutically acceptable salt thereof, wherein, R₄ is selected from the group consisting of: hydrogen, C₁₋₃ alkyl, C₁₋₃ haloalkyl, C₃₋₆ saturated or partially unsaturated carbocyclyl, 3-10 membered saturated or partially unsaturated heterocyclyl.
In another preferred embodiment, R₄ is hydrogen or methyl.

5. The compound of claim 1, a deuterated compound thereof, or a pharmaceutically acceptable salt thereof, wherein, R₅, R₆, R₇, R₈, and R₉ are each independently selected from the group consisting of: hydrogen, halogen, hydroxyl, carboxyl, C₁₋₃ alkyl, C₁₋₃ alkoxy, C₁₋₃ haloalkyl, and C₁₋₃ haloalkoxy.
In another preferred embodiment, R₅, R₆, R₇, R₈, and R₉ are each independently selected from the group consisting of: hydrogen, fluorine, chlorine.

6. The compound of claim 1, a deuterated compound thereof, or a pharmaceutically acceptable salt thereof, wherein, the compound is selected from the group consisting of:

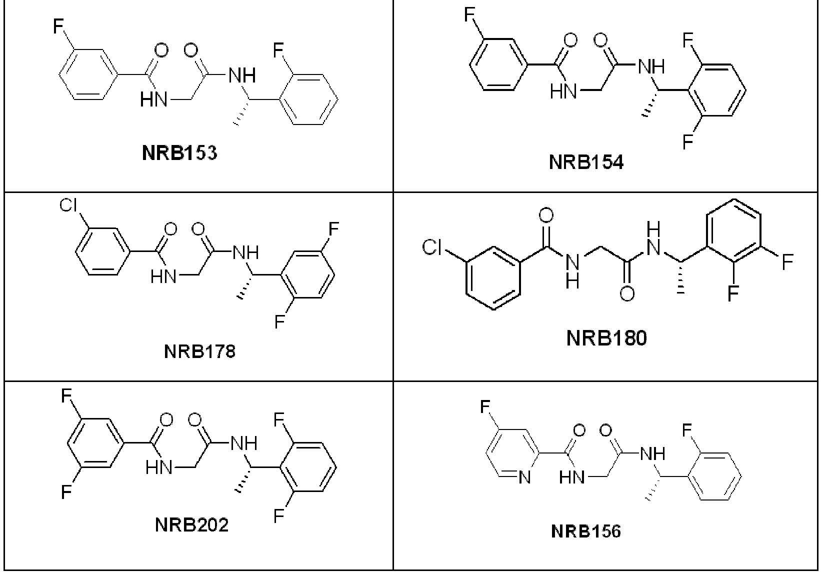

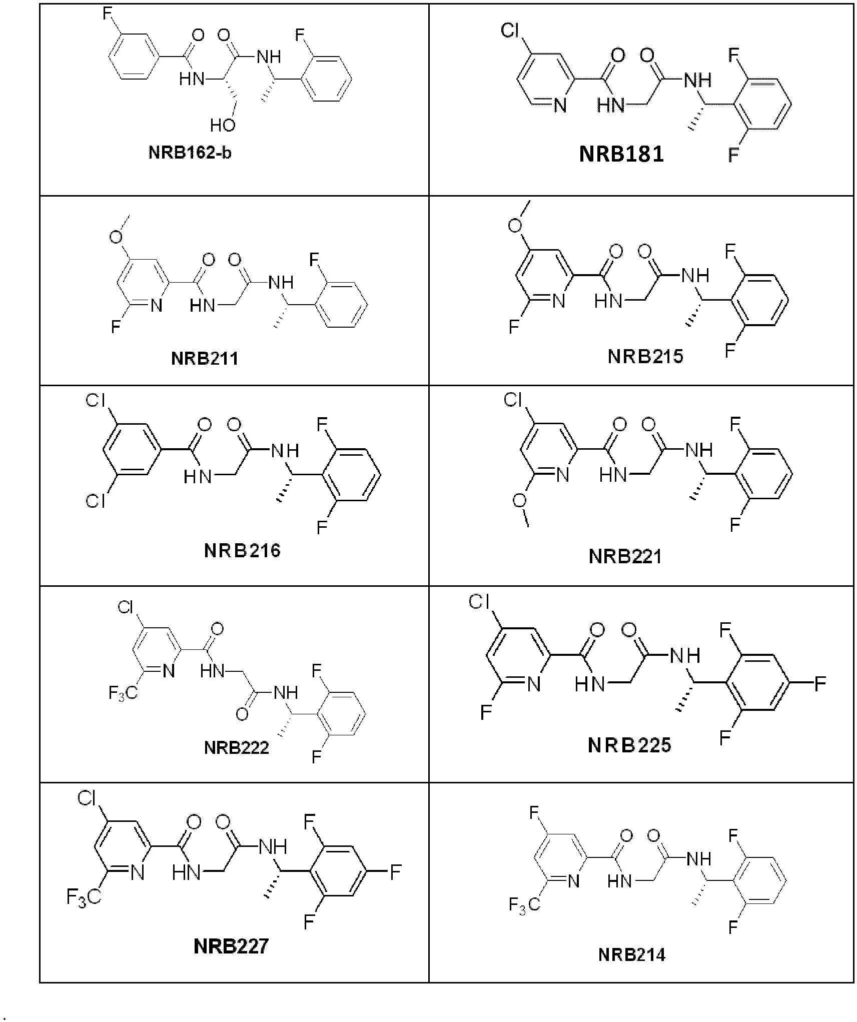

7. The method for preparing a compound of any one of claims 1-6 or a pharmaceutically acceptable salt thereof, comprising the steps of: 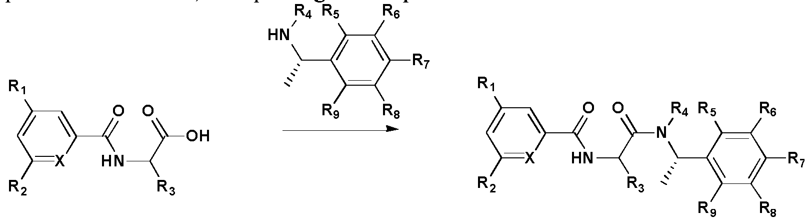 or 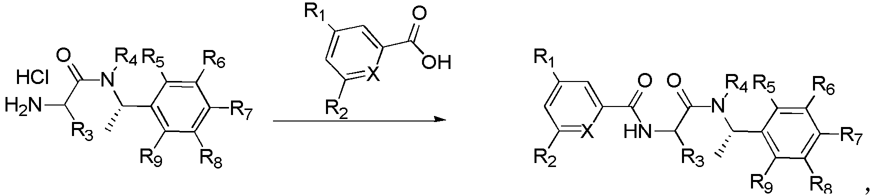 wherein, the definitions of X, R₁, R₂, R₃, _{R4,} R₅, R₆, R₇, _{R8,} and R₉ are ₐₛ described in claim 1

8. A pharmaceutical composition, comprising (1) a compound of any one of claims 1-6, a deuterated compound thereof, or a pharmaceutically acceptable salt thereof; and (2) pharmaceutically acceptable carriers.

9. The preparation method of the pharmaceutical composition of claim 8, comprising the steps of: mixing compound of any one of claims 1-6, a deuterated compound thereof, or a pharmaceutically acceptable salt thereof, with a pharmaceutically acceptable carrier to form a drug mixture.

10. Use of the compound of any one of claims 1-6, a deuterated compound thereof, or a pharmaceutically acceptable salt thereof, or the pharmaceutical composition of claim 8, for the preparation of a medicament for treating or preventing a GPR139 receptor-associated disorders.
In another preferred embodiment, the GPR139 receptor-associated disorders comprises anxiety disorders, depression, mania, Parkinson's syndrome, drug and alcohol abuse, schizophrenia, autism, seizure, and epilepsy.
